(19) Europäisches Patentamt
European Patent Office
Office européen des brevets

(11) Numéro de publication: **0 263 740**
**A1**

(12) **DEMANDE DE BREVET EUROPEEN**

(21) Numéro de dépôt: **87402142.1**

(22) Date de dépôt: **24.09.87**

(51) Int. Cl.4: **C 07 K 9/00**
C 07 K 15/18, A 61 K 37/10,
G 01 N 33/569, C 12 N 1/38

(30) Priorité: **26.09.86 FR 8613504**

(43) Date de publication de la demande:
**13.04.88 Bulletin 88/15**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75007 Paris (FR)**

(72) Inventeur: **Lebleu Bernard§Rés. le Jardin aux Fontaines B2**
**140, rue Pioch de Boutonnet**
**34000 Montpellier (FR)**

**Bayard Bernard**
**11, rue des Marroniers**
**34170 Castelnau Le Lez (FR)**

**Lemaitre Marc**
**4, rue des Cèdres JACOU**
**34170 Castelnau Le Nez (FR)**

(74) Mandataire: **Grosset-Fournier, Chantal Catherine et al**
**SC Ernest Gutmann/Yves Plasseraud 67 boulevard Haussmann**
**F-75008 Paris (FR)**

(54) **Nouveaux conjugués de couplage entre des séquences d'ARN ou d'ADN et une protéine, leur procédé de préparation et leur application biologique.**

(57) L'invention a pour objet de nouveaux conjugués de couplage de formule I suivante

(I)

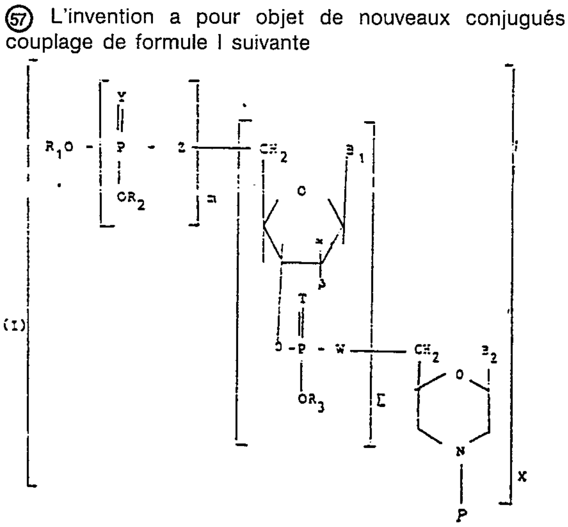

dans laquelle
- m est un nombre entier variant de 0 à 3,
- $R_1$, $R_2$, $R_3$ représentent un alcoyle de 1 à 10 atomes de carbone,
- Y et T représentent O ou S,
- Z et W représentent O, S, $CH_2$ ou NH,
- $B_1$ et $B_2$ représentent une base choisie parmi l'adénine, la guanine, la cytosine, la thymine ou l'uracile,
- (α, β) représente (H, H), (H, OH) ou (OH, H),
- Σ est un nombre entier supérieur ou égal à 7,
- X est un nombre entier compris de 1 à 5,
- P est un enchaînement polypeptidique.

Application à la préparation de réactifs biologiques.

**EP 0 263 740 A1**

NOUVEAUX CONJUGUES DE COUPLAGE ENTRE DES SEQUENCES D'ARN OU D'ADN ET UNE PROTEINE, LEUR PROCEDE DE PREPARATION ET LEUR APPLICATION BIOLOGIQUE

L'invention a pour objet de nouveaux conjugués de couplage entre des séquences d'ARN ou d'ADN et une protéine, leur procédé de préparation et leur application biologique, notamment antivirale.

On sait que des séquences d'oligonucléotides (en série ribo- ou désoxyribo-) complémentaires de régions convenablement choisies d'un ARN messager ou bien d'intermédiaires dans sa maturation, peuvent s'hybrider avec le susdit ARN messager par appariement de bases complémentaires, et donc bloquer l'expression de l'ARN messager en question. De telles séquences sont appelées séquences "anti-sens" (GREEN, P.J., PINES, O. and INOUYE, M. (1986). Ann. Rev. Biochem. 55, 569-597 ; IZANT, J.G. and WEINTRAUB, H. (1984). Cell 36, 1007-1015).

Ces séquences anti-sens ont été générées par transcription de séquences insérées selon une orientation inversée, à partir de promoteurs variés sur des plasmides recombinants introduits dans des cellules intactes par des techniques de transfection (COLEMAN, J., GREEN, P.J. and INOUYE, M. (1984). Cell, 37, 429-436 ; IZANT, J.G. and WEINTRAUB, H. (1985). Science 229, 345-352) ou par micro-injection (IZANT, J.G. and WEINTRAUB, H. (1984). Cell 36, 1007-1015) ; MELTON, D.A. (1985). Proc. Natl. Acad. Sci. USA 82, 144-148 ; KAWASAKI, E.S. (1985). Nucleic Acids Res. 13, 4991-5004).

En utilisant une approche quelque peu différente, on a synthétisé des analogues d'oligonucléotides méthylphosphonate non ioniques, qui possèdent une résistance accrue à la dégradation nucléolytique et pénètrent la membrane plasmique de cellules en culture, tout en conservant leur capacité à s'hybrider spécifiquement à des séquences d'ADN ou d'ARN complémentaires, (TS'O, P.O.P., MILLER, P.S. and GREENE, J.J. (1983) in Development of Target-Oriented Anticancer Drugs, eds. Cheng, Y.C., et al. (Raven Press, New-York), pp. 189-206 ; MILLER, P.S., AGRIS, C.H., AURELIAN, L., BLAKE, K.R., MURAKAMI, A., REDDY, M.P., SPITZ, S.A. and TS'O, P.O.P. (1985). Biochimie 67, 769-776).

Ces conjugués se sont révélés être actifs in vitro vis-à-vis de l'inhibition virale, mais à des concentrations de l'ordre de la centaine de micromole (150 µmoles ou 75 µmoles), ce qui semble être élevé pour une éventuelle utilisation en thérapeutique. De plus, la faible taille des oligonucléotides ( moins de 10 unitées nucléotidiques) internalisés par cette méthode rend la spécificité de reconnaissance et la stabilité d'hybridations faibles.

D'autres travaux ont été effectués en utilisant des oligodéoxynucléotides conjugués à l'acridine pour réguler l'expression de gènes viraux dans des extraits cellulaires (TOULME J.J. KRISCH, H.M., LOREAU, N., THUONG, N.T. and HELENE, (1986). Proc. Natl. Acad. Sci. USA, 83, 1227-1231 ; HELENE, C., MONTERAY-GARESTIER , T., SAISON, T., TAKASUGI, M., TOULME, J.J., ASSELINE, V., LANCELOT, G., MAURIZOT, J.C., TOULME, F. and THUONG, N.T. (1985). Biochimie 67, 777-783).

Mais, ces conjugués se sont révélés actifs in vitro à des concentrations de l'ordre de la centaine de micromoles, ce qui là encore semble être élevé pour une éventuelle utilisation en thérapeutique. L'efficacité avec laquelle ils permettent l'internalisation des séquences oligonucléotidiques conjuguées est mal documentée.

Par ailleurs, ces conjugués qui sont des composés intercalants, peuvent entraîner des risques de mutagénèse, en cas de destabilisation.

On a par ailleurs déjà synthétisé des conjugués covalents, d'une part entre des oligo et polynucléotides, en modifiant la séquence d 'ADN au niveau du phosphate 5' terminal des nucléotides impliqués et, d'autre part, entre des amines, des peptides et des protéines.

Des essais effectués en traitant un ADN plasmidique par la carbodiimide, ont montré que la modification apportée à la partie nucléique n'abolit pas la résistance du plasmide en question à la kanamycine. Mais, aucun essai biologique n'a été effectué relativement à l'internalisation de ces conjugués covalents à l'intérieur des cellules, ni à fortiori relativement à leurs éventuelles propriétés biologiques, notamment antivirales (BARBARA C.F. CHUS, GEOFFREY M. WAHL and LESLIE E. ORGEL (1983), Nucleic Acid Research 11, 6513).

On a également conjuqé l'$\alpha_2$ macroglobuline à des séquences d'ADN et le procédé utilisé est tel que le couplage a lieu sur des résidus de guanine non appariés, qui ont été introduits à l'extrémité de l'ADN.

Mais aucun essai biologique n'a été effectué relativement à l'internalisation du conjugué à l'intérieur des cellules, ni à fortiori relativement à leurs éventuelles propriétés biologiques, notamment antivirales (SHEUE-YANN CHENG, GLENN T. MERLINO and IRA H. PASTAN, (1983), Nucleic Acid Research 11, 659).

D'autres travaux ont porté sur la préparation de conjugués non covalents obtenus entre le sel d'ammonium quaternaire formé à partir d'une fonction carboxyle d'une protéine et les groupes phosphate d'une séquence d'ADN, mais aucun essai biologique n'a été effectué ni vis-à-vis de l'internalisation, ni a fortiori vis-à-vis des éventuelles propriétés biologiques, notamment antivirales (B. HUCKETT, HASHA GORDHAN, R. HAWTREY, N. MOODLEY, M. ARIATTI and A. HAWTREY (1986), Biochemical Pharmacology 35, nº 8, pp. 1249-1257).

Aucun des conjugués d'ADN obtenus à ce jour ne permet, d'une part, la reconnaissance d'une cellule cible vers laquelle on souhaite les diriger et, d'autre part, l'internalisation dans ladite cellule cible, à des concentrations susceptibles d'envisager leur utilisation dans le traitement thérapeutique, notamment des affections virales.

L'invention a pour objet de nouveaux conjugués de couplage entre une séquence d'ADN ou d'ARN et une protéine, susceptibles d'être internalisés dans des cellules cibles.

L'invention a pour objet de nouveaux conjugués de couplage susceptibles de délivrer des séquences antisens dans des cellules intactes.

Un autre aspect de l'invention est de fournir de nouveaux composés de couplage susceptibles d'être internalisés par les cellules, c'est-à-dire de traverser efficacement la membrane plasmique des cellules, tout en gardant leur activité biologique.

L'un des autres aspects de l'invention est de proposer de nouveaux conjugués de couplage qui peuvent être internalisés par la cellule, délivrer une séquence antisens et inhiber ainsi l'expression d'un gène, notamment d'un gène viral.

L'un des autres aspects de l'invention, est de proposer de nouveaux conjugués de couplage susceptibles d'être internalisés par une cellule cible, d'inhiber l'expression d'un gène particulier et éventuellement susceptibles de reconnaitre grâce è l'un des éléments des conjugués, une cellule cible de façon spécifique.

Un autre aspect de l'invention est de fournir de nouveaux composés de couplage susceptibles de réagir spécifiquement avec des récepteurs particuliers de la surface membranaire de la cellule, d'être internalisés par les cellules possédant lesdits récepteurs et d'exercer, à l'intérieur de ce cellules, leur activité biologique.

L'un des autres aspects de l'invention est de proposer de nouveaux conjugués de couplage susceptibles de présenter une activité antivirale à des doses compatibles avec un traitement thérapeutique.

Ces différents aspects de l'invention sont obtenus par de nouveaux conjugués de couplage

- dont une partie de la structure moléculaire correspond à une séquence d'ADN ou d'ARN dans laquelle les bases sont non modifiées, et dont la première unité nucléosidique comporte ou non, par l'intermédiaire de son carbone en 5', un nombre variable de groupes phosphate, l'un au moins des atomes d'oxygène relié uniquement au phosphore des groupes phosphate et n'intervenant pas dans la liaison entre deux groupes phosphate, peut être remplacée par un atome de soufre,

et/ou au moins l'une des liaisons entre deux groupes phosphates adjacents peut comporter un atome de soufre, ou un groupe NH, ou un groupe $CF_2$ ou un groupe $CH_2$,

et/ou la liaison entre le dernier groupe phosphate relié à la première unité nucléosidique de la séquence d'ADN ou d'ARN peut comporter un atome de soufre, ou un groupe NH, ou un groupe $CF_2$ ou un groupe $CH_2$,

et/ou la liaison 3'-5' phosphonate qui relie deux unités nucléosidiques adjacente est telle que l'oxygène, relié uniquement au phosphore, et n'entrant pas dans la liaison directe entre les deux unités nucléosidiques adjacente est remplacé pare un atome de soufre, ou bien est tel que l'oxygène qui relie le phosphore à l'extrémité 5' de l'une des unités nucléosidiques est remplacé par ou un groupe NH, ou un groupe $CF_2$ ou un groupe $CH_2$,

- et dont l'autre partie de la structure moléculaire correspond à celle d'un enchaînement peptidique qui est relié au groupe phosphate en 3' de la dernière unité nucléosidique de la séquence d'ADN ou d'ARN, par l'intermédiaire d'un bras de couplage, lequel bras de couplage provient avantageusement d'une séquence latérale, portée par l'enchaînement peptidique, dont l'extrémité est constituée par un groupe aminé, notamment $NH_2$.

Dans une classe préférée d'oligonucléotides selon l'invention, la première unité nucléosidique est liée à un ou plusieurs groups phosphate.

De préférence, le nombre de ces groupes phosphate est de 1.

Dans une classe préférée de composés de couplage de l'invention, la première unité nucléosidique est liée aux groupes phosphate suivants :

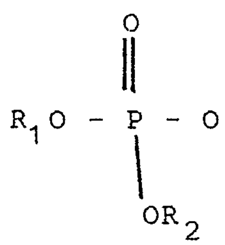

dans lesquels $R_1$ et $R_2$ représentent indépendamment les uns des autres :

- un atome d'hydrogène,
- un radical alcoyle ayant de 1 à 10 atomes de carbone, de préférence 1 ou 2 atomes de carbone,
- un radical éthyle substitué en bêta par un groupe cyano, aryle ou arylsulfonyle,
- un radical trihalogénoéthyle.

Dans une classe préférée de composés de couplage selon l'invention, la liaison 3' → 5' reliant deux unités nucléosidiques et comprenant au moins un atome de phosphore est une liaison phosphodiester, une liaison phosphotriester ou une liaison alcoylphosphonate.

La liaison 3' → 5' phsophodiester qui relie deux unités nucléosidiques adjacents dans les composés de couplages selon l'invention peut être représentée comme suit :

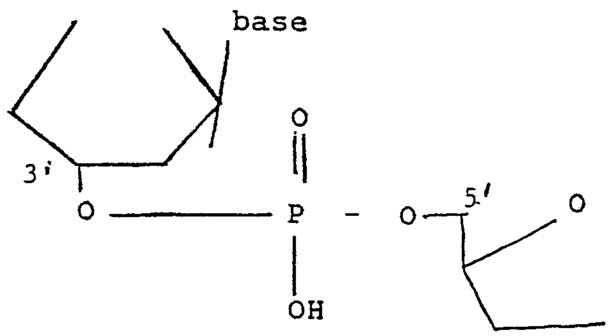

La liaison 3' → 5' phosphotriester qui relie deux unités nucléosidiques adjacents dans les composés de couplage de l'invention peut être représentée comme suit

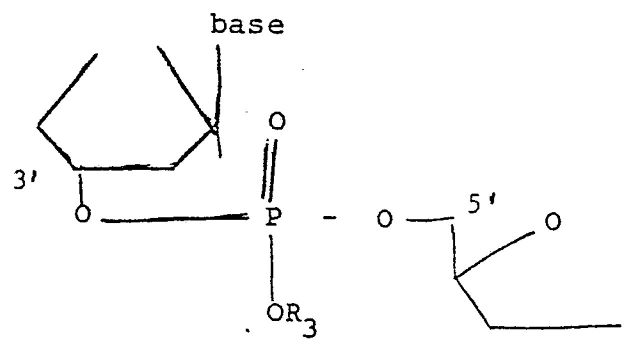

dans laquelle $R_3$ représente
- un radical alcoyle ayant de 1 à 4 atomes de carbone, en particulier un méthyle,
- un radical éthyle substitué en bêta par un groupe cyano, aryle ou arylsulfonyle,
- un radical trihalogénoéthyle.

La liaison 3' → 5' phosphonate qui relie deux unités nucléosidiques adjacentes dans les composés de couplages elon l'invention peut être représentée comme suit :

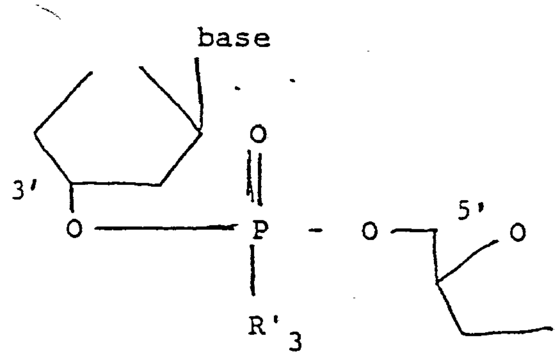

dans laquelle $R'_3$ peut représenter un alcoyle ayant de 1 à 4 atomes de carbone, en particulier le méthyle.

En ce qui concerne les bases des oligonucléotides, leur nombre est égal à la valeur du nombre d'oligonucléotides et chacune a une signification telle que l'enchaînement des oligonucléotides résultants correspond à une séquence d'ADN ou d'ARN. En d'autres termes, lorsque l'enchaînement oligonucléotidique est un enchaînement d'ARN, la base peut représenter A, U, C ou G, et lorsqu'il s'agit d'un enchaînement d'ADN, la base peut représenter A, T, C ou G.

La séquence d'ADN ou d'ARN entrant dans la constitution des conjugués de l'invention correspond à la séquence complémentaire d'une séquence oligonucléotidique cible (ci-après également désignée par séquence oligonucléotidique antisens), avantageusement à une séquence d'ADN ou d'ARN complémentaire d'un ARN messager.

La séquence d'ADN ou d'ARN peut également correspondre à la séquence complémentaire d'un ARN messager et en particulier à la séquence complémentaire d'une séquence de fixation aux ribosomes, d'un site d'épissage et, de manière générale, à toute séquence complémentaire d'ADN ou d'ARN critique au mécanisme d'expression du (ou des ) gène(s) visé(s), telle que le site de reconnaissance de RNA polymérase virale...

La séquence d'ADN ou d'ARN entrant dans la constitution des conjugués de couplage de l'invention peut

être telle que l'ordre des bases est modifié par rapport à celui de la séquence complémentaire de la séquence oligonucléotidique cible, notamment la séquence complémentaire d'un ARN messager et/ou les bases sont éventuellement substituées dans la mesure où l'hybridation entre la susdite séquence oligonucléotidique complémentaire de la séquence oligonucléotidique cible, notamment la séquence d'ARN ou d'ADN et la séquence oligonucléotidique cible, notamment la séquence d'ARN messager reste caractéristique et non équivoque.

Selon un mode de réalisation préféré de l'invention, les conjugués de couplage sont tels que l'enchaînement des oligonucléotides correspond à la séquence complémentaire de l'extrémité 5′ d'un ARN messager.

Les conjugués de l'invention présentent ainsi la propriété avantageuse que la structure des différentes bases des séquences d'ARN ou d'ADN n'est pas modifiée ; ceci vise à maintenir optimales les caractéristiques de reconnaissance entre la séquence oligonucléotidique entrant dans la constitution des conjugués de l'invention et la séquence oligonucléotidique cible.

Le nombre d'oligonucléotides entrant dans la constitution des conjugués de couplage de l'invention doit être égal ou supérieur à 7. En effet, en dessous de cette valeur peuvent surgir des problèmes de mauvaise hybridation du conjugué de l'invention vis-à-vis de la séquence oligonucléotidique cible.

De plus, en dessous de cette valeur, la spécificité du conjugué de couplage de l'invention risque de ne plus être suffisante.

Le nombre d'unités nucléosidiques entrant dans la constitution des composés de couplage de l'invention n'est pas limité dans les valeurs supérieures, sous réserve que les conjugués de couplage obtenus puissent être internalisés.

Le nombre d'unités nucléosidiques peut rapidement être limité dans la mesure où l'augmentation de ce nombre et la synthèse correspondante plus difficiles (lorsque les conjugués sont préparés par voie de synthèse chimique) ne seraient pas supportées par une augmentation suffisante de l'activité.

Le nombre d'unités nucléosidiques devrait être chosi de façon à ce que le poids moléculaire de l'oligonucléotide entrant dans la constitution du composé de couplage selon l'invention soit de préférence compris de 1 500 à 25 000 daltons.

Dans une classe préférée de conjugués de couplage selon l'invention, le nombre d'oligonucléotides n'est pas supérieur à 50, et est avantageusement de 7 à 20, et notamment de 12 à 18.

En ce qui concerne la dernière base de la séquence d'ADN ou d'ARN entrant dans la constitution des conjugués de couplage de l'invention, elle peut ou non intervenir dans l'hybridation du reste du conjugué de l'invention avec la séquence oligonucléotidique cible avec lequel le conjugué de l'invention doit s'hybrider.

La signification de la dernière base peut dépendre du mode de synthèse utilisé, et compte tenu des produits intermédiaires facilement accessibles, elle représente avantageusement la cytidine.

De façon générale, lorsque le conjugé de l'invention sera un conjugué d'ADN, la dernière base pourra ne pas intervenir dans l'hybridation du conjugué de couplage de l'invention avec la séquence oligonucléotidique cible.

Lorsque le conjugué de l'invention est un conjugué d'ARN, la dernière base pourra intervenir dans l'hybridation du conjugué avec la séquence oligonucléotidique cible.

En ce qui concerne les radicaux $R_1$, $R_2$, $R_3$, déjà représentés ci-dessus, ils participent à l'augmentation de la stabilité des conjugués de l'invention, lorsqu'ils représentent un groupe alcoyle de 1 à 10 atomes de carbone.

L'enchaînement polypeptidique P peut être naturel ou synthétique, mais doit présenter des caractéristiques telles qu'il puisse être internalisé dans la cellule cible et peut présenter des caractéristiques telles qu'il puisse être avantageusement reconnu spécifiquement par des récepteurs de la membrane cellulaire d'une cellule cible.

L'enchaînement polypeptidique selon l'invention comporte au moins un groupement accessible capable de réagir avec un groupe fonctionnel, notamment une fonction aldéhyde des oligonucléotides avec lesquels on souhaite effectuer le couplage entre le susdit oligonucléotide et l'enchaînement polypeptidique, et ce groupe fonctionnel peut être avantageusement une séquence latérale dont l'extrémité est constituée par un groupe aminé, notamment $NH_2$, qui est accessible.

On désigne par groupe aminé d'une séquence latérale le groupe aminé d'un polypeptide qui n'entre pas dans les liaisons $-\underset{\underset{O}{\|}}{C}-NH$ peptidiques.

Le couplage entre le polypeptide et la séquence d'ADN ou d'ARN peut également avoir lieu par l'intermédiaire du $NH_2$ terminal du polypeptide.

L'enchaînement polypeptidique entrant dans la constitution des composés de couplage selon l'invention doit être non toxique aux concentrations utilisées vis-à-vis des cellules dans lesquelles les composés de couplage sont introduits.

De façon avantageuse, la séquence latérale dont l'extrémité est constituée par un groupe $NH_2$ appartient au résidu lysyle.

L'enchaînement polypeptidique peut également avantageusement comporter des résidus arginyle, étant donné qu'il présente une séquence latérale terminée par un groupe $NH_2$.

L'enchaînement polypeptidique entrant dans la constitution des composés de couplage selon l'invention comporte avantageusement au moins 5 acides aminés.

Dans le cas de polypeptides synthétiques, on a avantageusement recours à des polypeptides de 5 à environ

100 acides aminés, et de préférence de 60 à 100 acides aminés.

L'enchaînement polypeptidique doit avoir un poids moléculaire suffisant notamment pour être reconnu par les récepteurs spécifiques de la surface membranaire des cellules.

L'enchaînement polypeptidique entrant dans la constitution des composés de couplage selon l'invention a avantageusement un poids moléculaire moyen au moins d'environ 1 000.

L'enchaînement polypeptidique entrant dans la constitution des composés de couplage selon l'invention est avantageusement constitué par la polyarginine, et de préférence par la polylysine.

La polylysine entrant dans les composés de couplage de l'invention comporte avantageusement 5 à environ 100 résidus lysyle.

La polylysine entrant dans les composés de couplage selon l'invention a avantageusement un poids moléculaire moyen d'environ 5 000 à environ 40 000, notamment d'environ 10 000 à environ 20 000, et avantageusement d'environ 14 000.

Les composés de couplage de l'invention sont tels qu'il y ait au moins une molécule d'oligonucléotides fixée sur le même enchaînement polypeptidique, par l'intermédiaire d'une séquence latérale terminée par un groupe aminé, notamment $NH_2$, appartenant à l'enchaînement peptidique.

De façon avantageuse, le nombre de molécules d'oligonucléotides par rapport à une molécule d'enchaînement polypeptidique est égal à 1, mais sans limite supérieure théorique.

En pratique, le nombre de molécules d'oligonucléotides couplées à une molécule polypeptidique est limité par le nombre de $NH_2$ disponibles sur l'enchaînement polypeptidique, c'est-à-dire par le nombre de séquences latérales terminées par des groupes aminés, notamment $NH_2$, augmenté du $NH_2$ terminal, par le rendement de la réaction de couplage, lequel peut être modulé entre autres par les quantités respectives d'oligonucléotide et de polypeptide mises en oeuvre, ainsi que par l'encombrement maximal au-delà duquel le maintien des propriétés d'internalisation et de re connaissance éventuelle d'un récepteur membranaire n'est plus possible.

L'invention a également pour objet de nouveaux composés de couplage, dans lesquels une partie de la structure moléculaire correspond à une séquence d'ADN ou d'ARN dans lesquels l'autre partie de la structure moléculaire est constituée par une enchaînement polypeptidique dans lequel des groupes $NH_2$ ont été fixés sur les groupes carboxyliques.

Les groupes carboxyliques d'un polypeptide sur lesquels on fixe un groupe $NH_2$ sont :

- le groupe carboxylique terminal libre qui figure dans tout polypeptide ;
- et/ou les groupes carboxyliques libres des résidus glutamyle et aspartyle.

On fixe par exemple le groupe $-NH_2$ sur un groupe $-COOH$, par l'intermédiaire de l'hydrazine $NH_2NH_2$, ce qui transforme $-\underset{\underset{O}{\|}}{C}-OH$ en $-\underset{\underset{O}{\|}}{C}-NH-NH_2$

Dans le cas où le groupe COOH sur lequel on a fixé le groupe $NH_2$ appartient à un résidu aspartyle, celui-ci devient un asparte hydrazide.

Dans le cas où le groupe COOH sur lequel on a fixé le groupe $NH_2$ apparteint à un résidu glutamyle, celui-ci devient un glutamate hydrazide.

On appellera "polypeptide hydrazide" le polypeptide dans lequel l'un au moins des groupes COOH a été transformé en $-\underset{\underset{O}{\|}}{C}-NH-NH_2$,

comme indiqué ci-dessus.

La présence de ces groupes $NH_2$ libres permet d'effectuer le couplage entre une séquence d'ADN ou d'ARN et le polypeptide hydrazide.

L'invention vise également les composés de couplage dans lesquels une partie de la structure moléculaire correspond à une séquence d'ADN ou d'ARN, et l'autre partie de la structure moléculaire correspond à un polypeptide qui comporte des séquences latérales terminées par un groupe $-NH_2$ et des groupes carboxyliques qui ont été transformés en $-\underset{\underset{O}{\|}}{C}-NH-NH_2$.

De tels polypeptides sont par exemple constitués par des asialoglycoprotéines, telles que l'asialofétine.

L'invention vise également les composés de couplage dans lesquels une partie de la structure moléculaire correspond aux oligonucléotides sus-définis et l'autre partie de la structure moléculaire correspond à une néoglycoprotéine, par exemple une néoglycoprotéine à mannose, telle que le mannose-polylysine qui est constitué par de la polylysine, sur laquelle a été fixée au moins une molécule de mannose.

L'invention concerne plus généralement les composés de couplage dans lesquels une partie de la structure moléculaire correspond aux oligonucléotides sus-définis, et l'autre partie de la structure moléculaire correspond à un vecteur polypeptidique approprié, tel que des anticorps vis-à-vis d'antigènes, de la surface cellulaire des hormones, des glycoprotéines, des chaînes B de toxines animales ou végétales, lequel vecteur peut être couplé aux oligonucléotides par l'intermédiaire du $-NH_2$ libre des séquences latérales du polypeptide ou bien par l'intermédiaire du $-NH_2$ des groupes $-\underset{\underset{O}{\|}}{C}-NH-NH_2$,

provenant de la réaction avec l'hydrazine du groupe carboxyle terminal du polypeptide ou des groupes carboxyle appartenant aux résidus aspartyle et ou glutamyle.

L'invention a pour objet de nouveaux conjugués de couplage de formule I

(I) 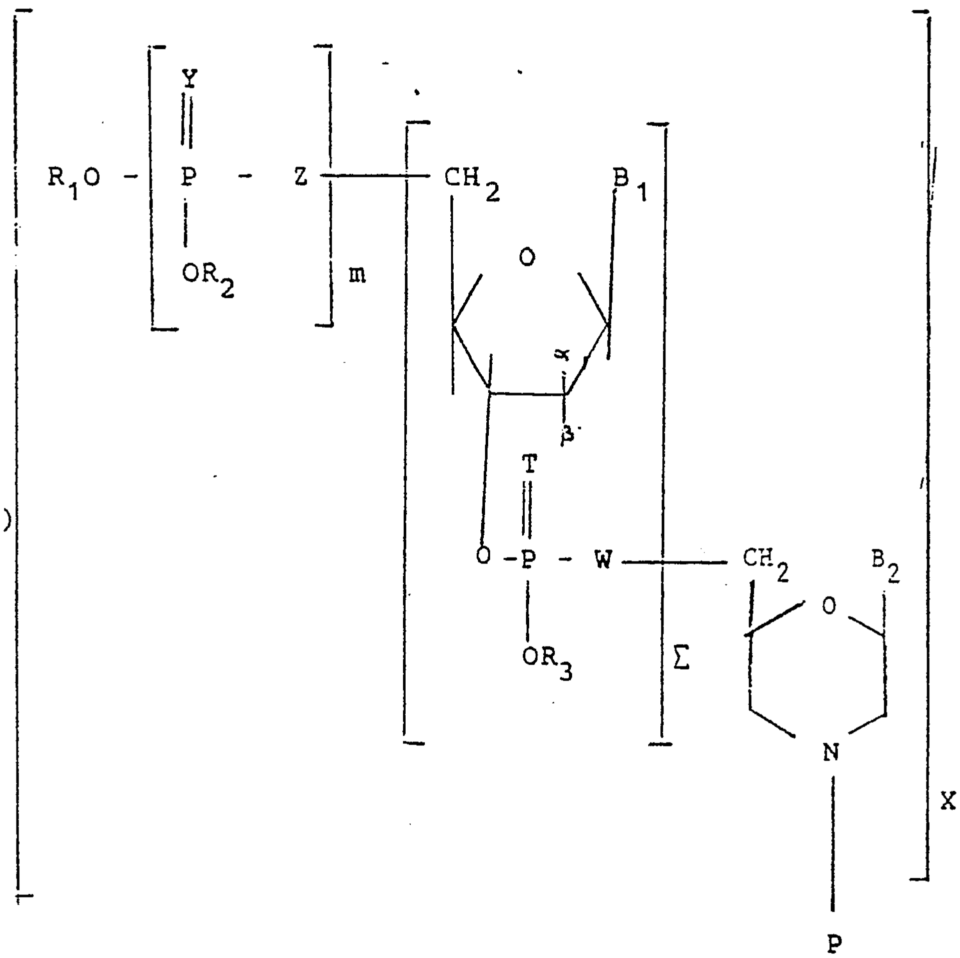

dans laquelle

- m est un nombre entier variant de 0 à 3, et représente de préférence 0 ou 1,
- $R_1$, $R_2$, $R_3$ sont identiques ou différents et représentent un alcoyle de 1 à 10 atomes de carbone, de préférence de 1 à 2 atomes de carbone, ou bien chacun des groupes $OR_1$, $OR_2$, $OR_3$ représente indépendamment l'un de l'autre 0−,
- Y et T, identiques ou différents, représentent O ou S,
- Z et W, identiques ou différents, représentent O, S $CH_2$, $CF_2$, ou NH,
- $B_1$ et $B_2$, identiques ou différents, représentent une base choisie parmi l'adénine (A), la guanine (G), la cytosine (C), la thymine (T) ou l'uracile (U), - (α, β) représente (H,H), (H, OH) ou (OH, H),
- Σ est un nombre entier supérieur ou égal à 7, avantageusement compris de 7 à 30, de préférence de 7 à 20, et notamment de 12 à 15,
- X est un nombre entier compris de 1 à 5, et avantageusement vaut 1,
- P est un enchaînement polypeptidique comportant au moins 5 acides aminés, naturels ou synthétiques, et l'atome d'azote appartenant au cycle

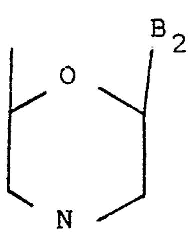

appartenant également à l'extrémité d'une séquence latérale du polypeptide P et /ou appartenant à un groupe $-\underset{\overset{\|}{O}}{C}-NH-N,$

provenant d'un groupe COOH modifié par l'hydrazine.

Selon un mode de réalisation avantageux, les conjugués de l'invention sont tels que l'enchaînement

peptidique P comporte au moins 5 acides aminés et l'atome d'azote appartenant au cycle

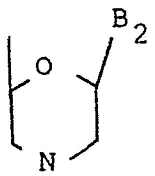

appartient également à l'extrémité d'une séquence latérale du polypeptide P.

Un groupe avantageux de conjugués de l'invention est constitué par ceux de formule suivante II :

(II)

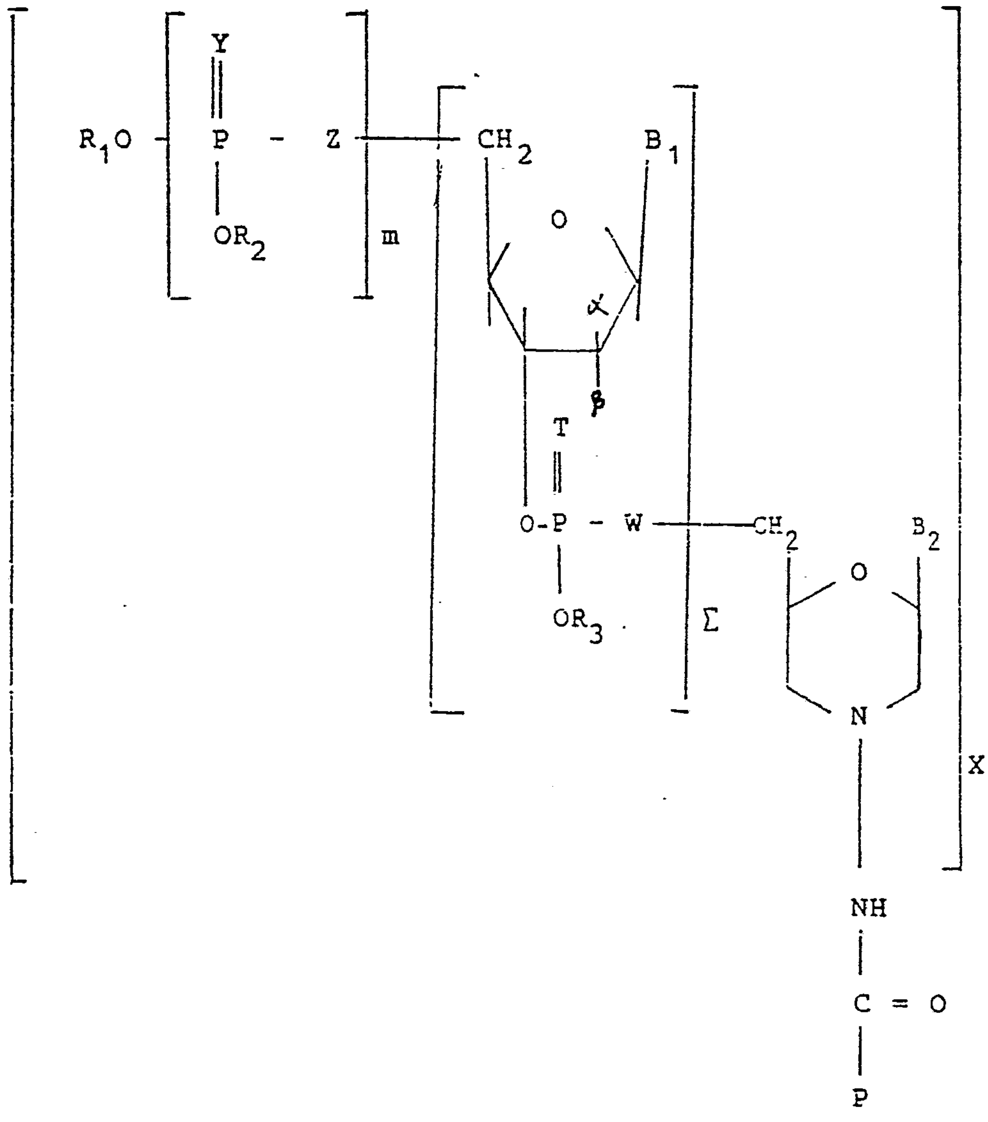

dans laquelle Y, Z, T, W, Σ, m, α, β, X, $R_1$, $R_2$, $R_3$, $B_1$, $B_2$ ont les significations définies ci-dessus, P représente un enchaînement polypeptidique, comportant au moins 5 acides aminés, et dans lequel le groupe $-\underset{\overset{\|}{O}}{C}-NH-N$ provient d'un groupe $-\underset{\overset{\|}{O}}{C}-OH$ terminal du polypeptide et/ou d'un groupe $-\underset{\overset{\|}{O}}{C}-OH$ libre d'un résidu aspartyle et/ou glutamyle.

Un autre groupe préféré des conjugués de l'invention est constitué par ceux de formule III

(III) 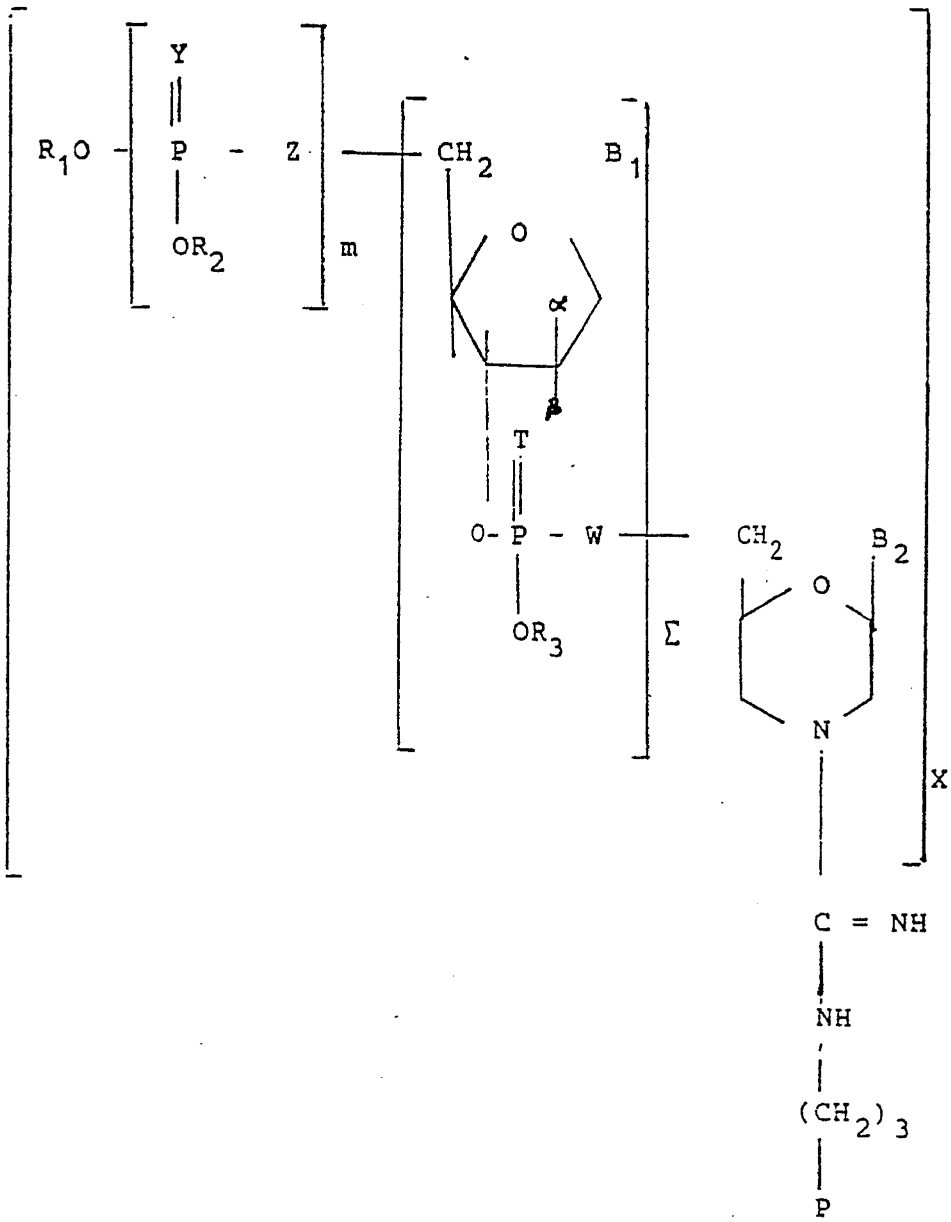

dans laquelle Y, Z, T, W, Σ, m, α, β, X, $R_1$, $R_2$, $R_3$, $B_1$, $B_2$ ont les significations définies ci-dessus, P est un enchaînement peptidique comportant au moins 5 acides aminés et le groupe
NH
-C - NH - $(CH_2)_3$ - appartenant à l'extrémité de la séquence latérale d'un résidu arginyle entrant dans la constitution de l'enchaînement polypeptidique P.

un autre groupe avantageux de conjugués de l'invention est constitué par ceux de formule IV suivante :

(IV) 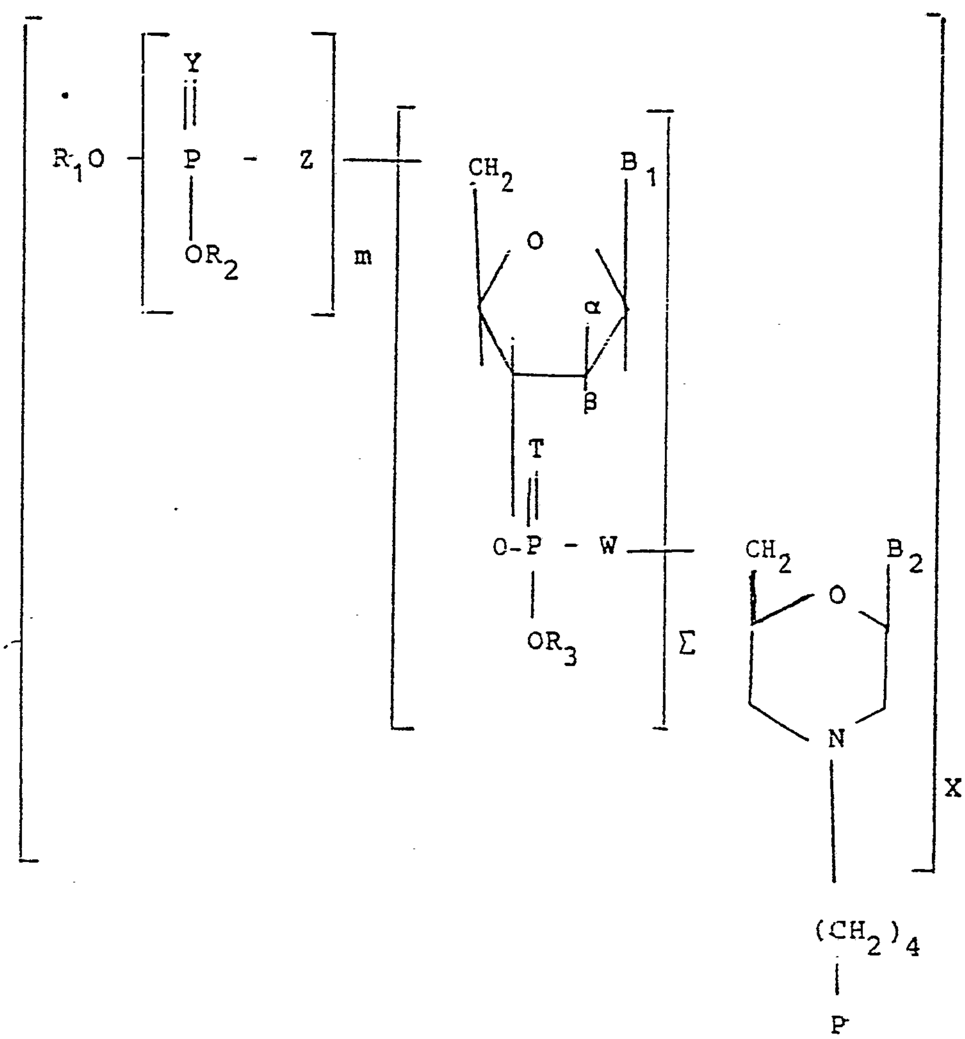

dans laquelle Y, Z, T, W, Σ, m, α, β, X, $R_1$, $R_2$, $R_3$, $B_1$, $B_2$ ont les significations définies ci-dessus et P est un enchaînement peptidique comportant au moinss 5 acides aminés et dans laquelle le groupe - $(CH_2)_4$ - appartient à la séquence latérale d'un résidu lysyle entrant dans la constitution de l'enchaînement polypeptidique P.

Un groupe avantageux de conjugués de couplage de l'invention est constitué par ceux de formule I, II, II ou IV dans lesquels l'un au moins des éléments Y ou Z est différent de l'oxygène.

Un autre groupe avantageux de conjugués de couplage de l'invention est constitué par ceux de formule I, II, III et dans lesquels Y, Z, T, W représentent tous l'oxygène.

Un autre groupe avantageux de conjugués de l'invention est constitué par ceux de formule IV, dans lesquels Y, Z, T et W représentent tous l'oxygène et répondent à la formule V suivante :

(V) 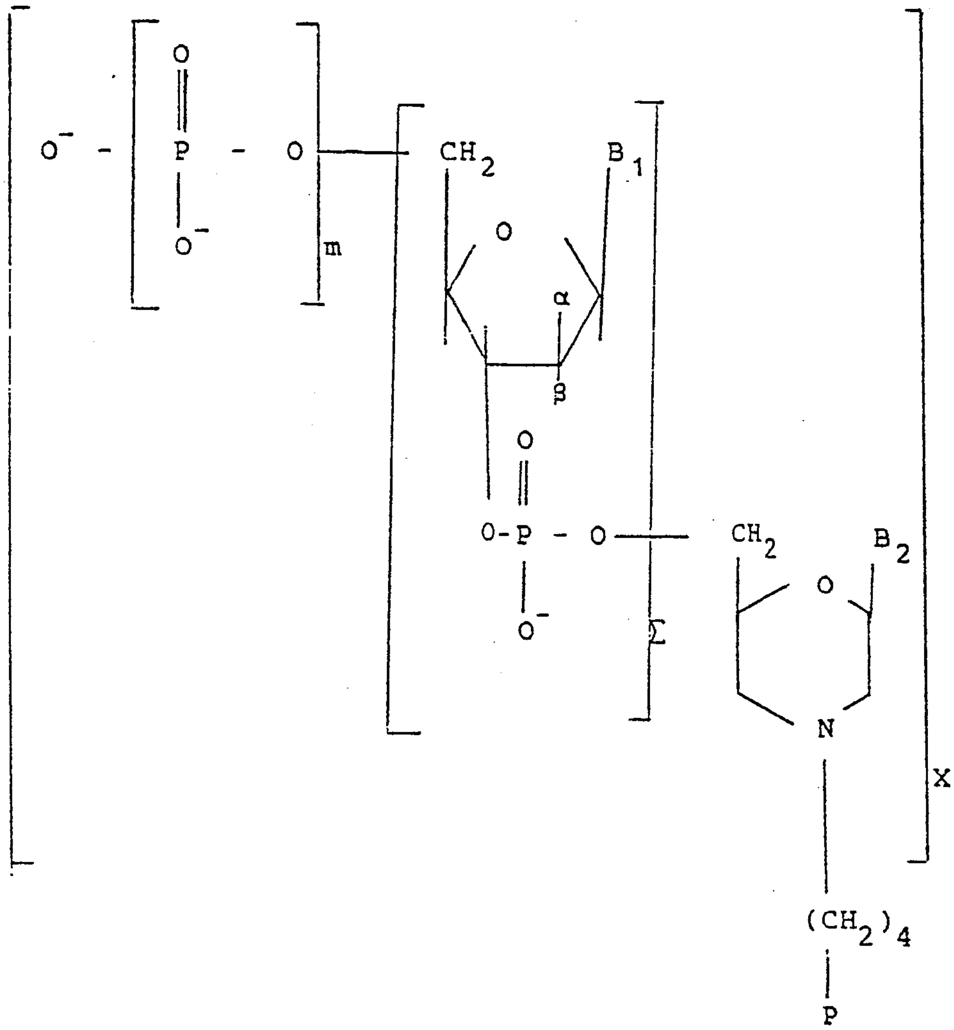

dans laquelle m, Σ, X, α, β, $B_1$, $B_2$ et P ont les significations indiquées ci-dessus.

Un autre groupe avantageux de conjugués de l'invention est constitué par ceux de formule I, II, III ou IV dans lesquels m vaut 0.

Un autre type avantageux de conjugués de l'invention est constitué par ceux de formule V dans lesquels m vaut 0 et répondent à la formule VI suivante :

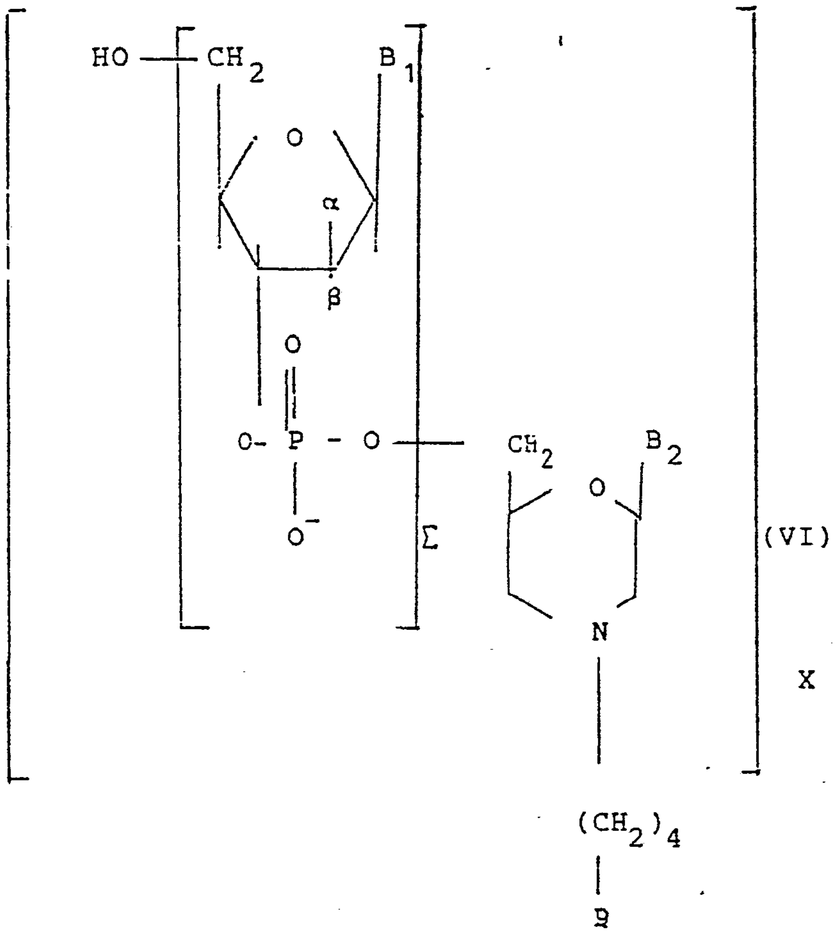

(VI)

dans laquelle $B_1$, $B_2$, α, β, Σ, X et P ont les significations indiquées ci-dessus.

Un groupe avantageux de conjugués de l'invention est constitué par ceux de formule I, II, III, IV dans lesquels le couple (α, β) représente (H, H), c'est-à-dire dans lesquels la séquence d'oligonucléotides est une séquence d'ADN.

Un groupe avantageux de conjugués de l'invention est constitué par ceux de formule I, II, III, IV dans lesquels le couple (α, β) représente (H, OH), c'est-à-dire dans lesquels la séquence d'oligonucléotides est une séquence d'ARN.

Un autre groupe avantageux de conjugués de l'invention est constitué par ceux de formule V, dans lesquels le couple (α, β) représente (H, H).

Ces conjugués répondent à la formule VII suivante :

(VII )

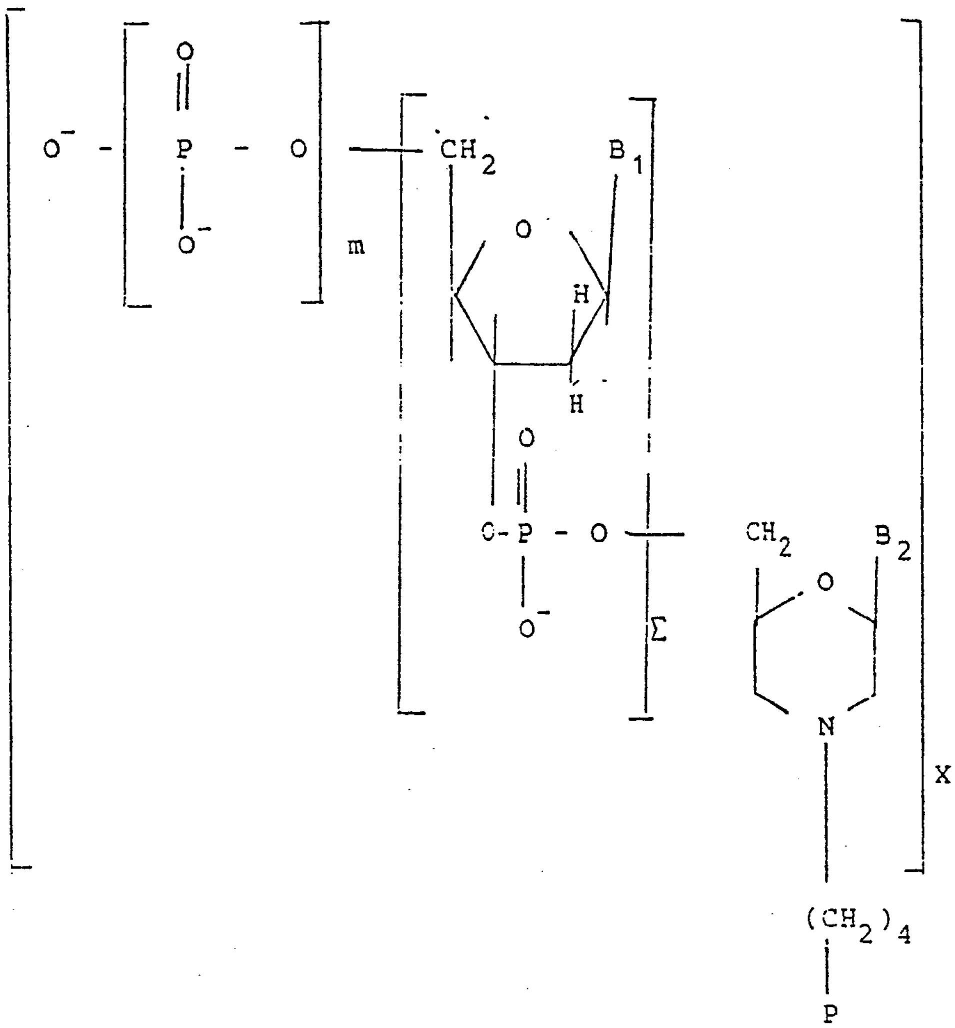

dans laquelle $B_1$, $B_2$, $\Sigma$, X, m et P ont les significations indiquées ci-dessus.

Un autre groupe avantageux de conjugués de l'invention est constitué par ceux de formule VI, dans lesquels le groupe ($\alpha$, $\beta$) représente (H, H).

Ces conjugués répondent à la formule (VIbis) suivante :

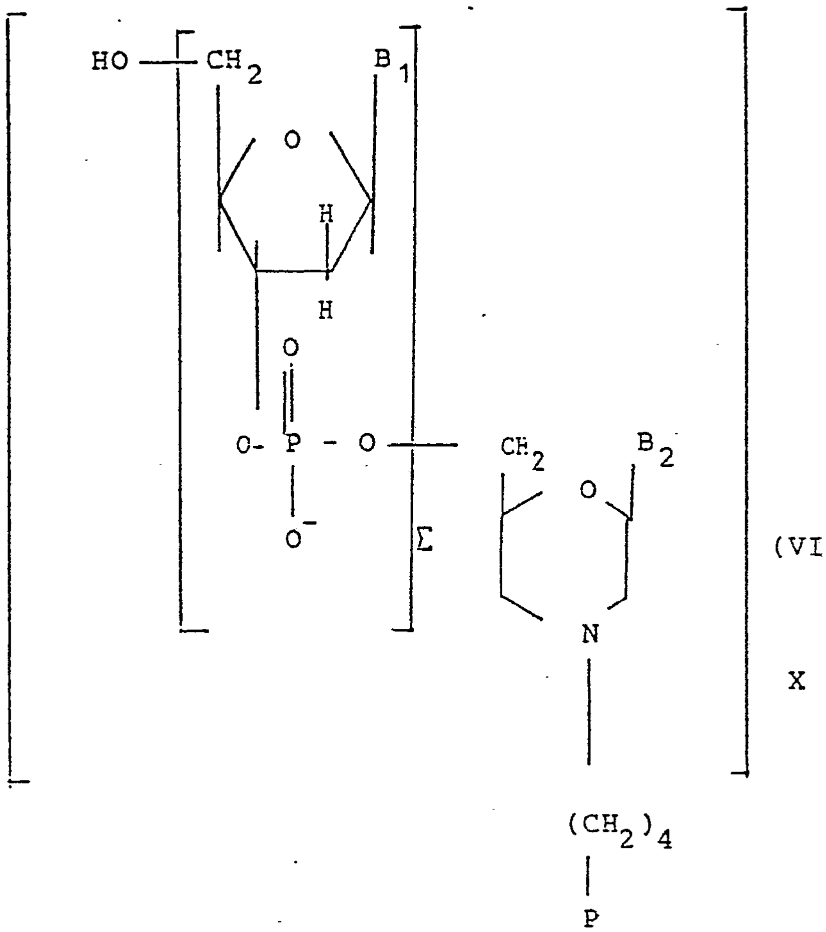

(VIbis)

dans laquelle $B_1$, $B_2$, $\Sigma$, X et P ont les significations indiquées précédemment.

Un autre groupe avantageux de conjugués de l'invention est constitué par ceux de formule V, dans lesquels le couple (α, β) représente (H, OH).

Ces conjugués répondent à la formule VIII suivante :

(VIII) 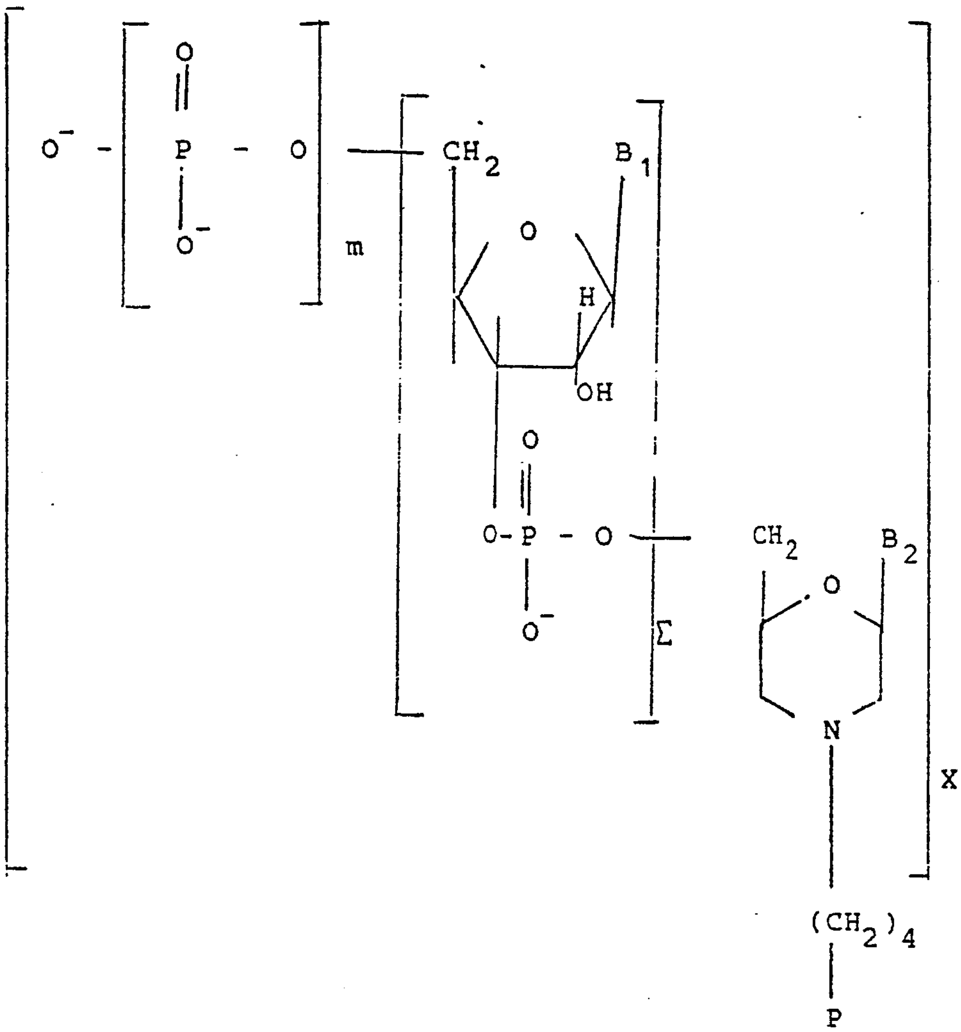

dans laquelle $B_1$, $B_2$, X, m, Σ et P ont les significations indiquées précédemment.

Un autre groupe avantageux de conjugués de l'invention est constitué par ceux de formule VI, dans lesquels le groupe (α, β) représente (H, OH).

Les conjugués répondent à la formule VIter suivante :

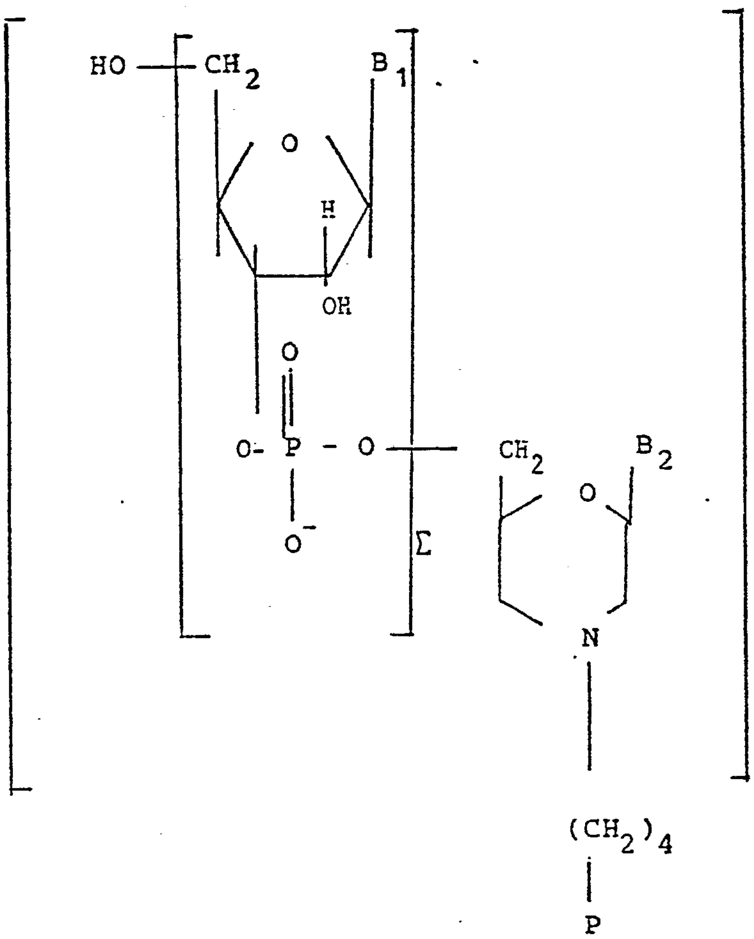

VIter)

dans laquelle $B_1$, $B_2$, α, β, Σ, X et P ont les significations indiquées précédemment.

Un autre groupe avantageux de conjugués de l'invention est constitué par ceux de formule I, II, III, IV dans lesquels le couple (α, β) représente (OH, H).

Un autre groupe avantageux de conjugués de l'invention est constitué par ceux de formule V, dans lesquels le couple (α, β) représente (OH, H).

Ces conjugués répondent à la formule IX suivante :

(IX) 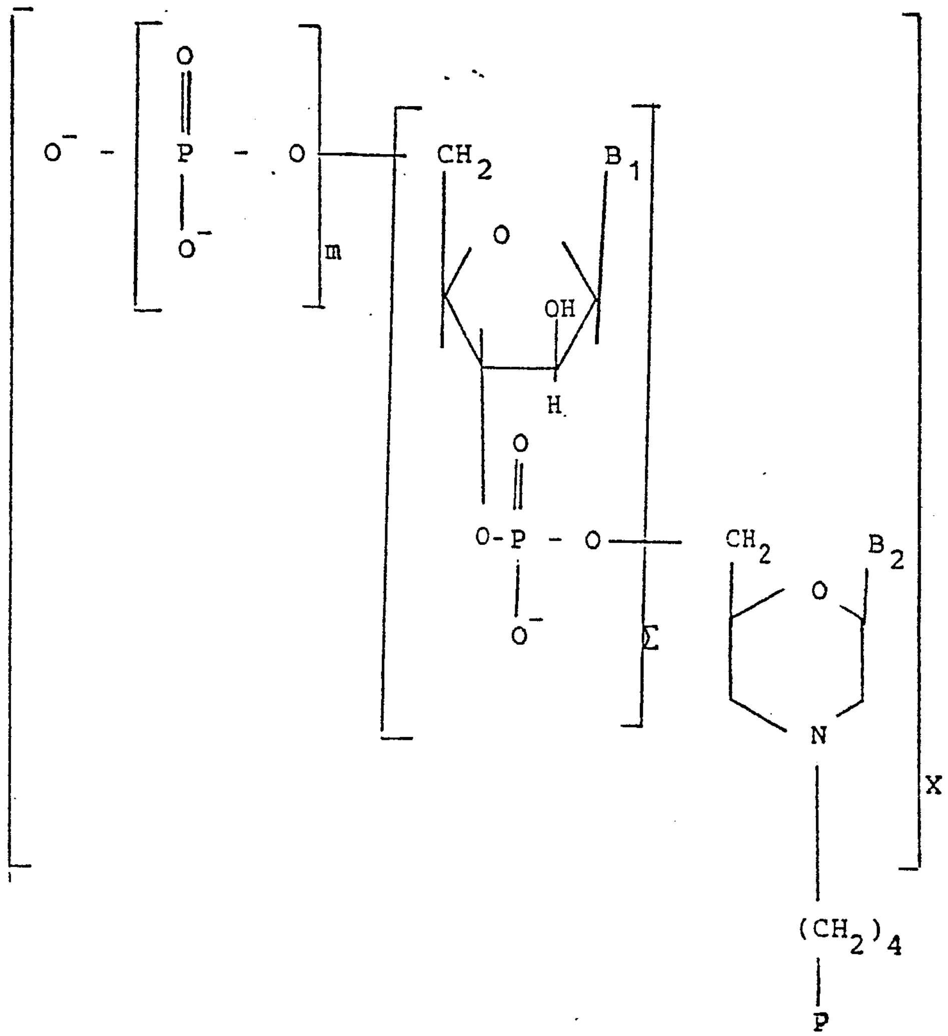

dans laquelle $B_1$, $B_2$, X, m, Σ et P ont les significations indiquées ci-dessus.

Un autre groupe avantageux de conjugués de l'invention est constitué par ceux de formule I, II, III, IV, VI, dans lesquels $B_2$ représente la cytidine.

Un autre groupe avantageux de conjugués de l'invention est constitué par ceux de formule V, dans lesquels $B_2$ représente la cytidine.

Ces conjugués répondent à la formule X suivante :

(XI')

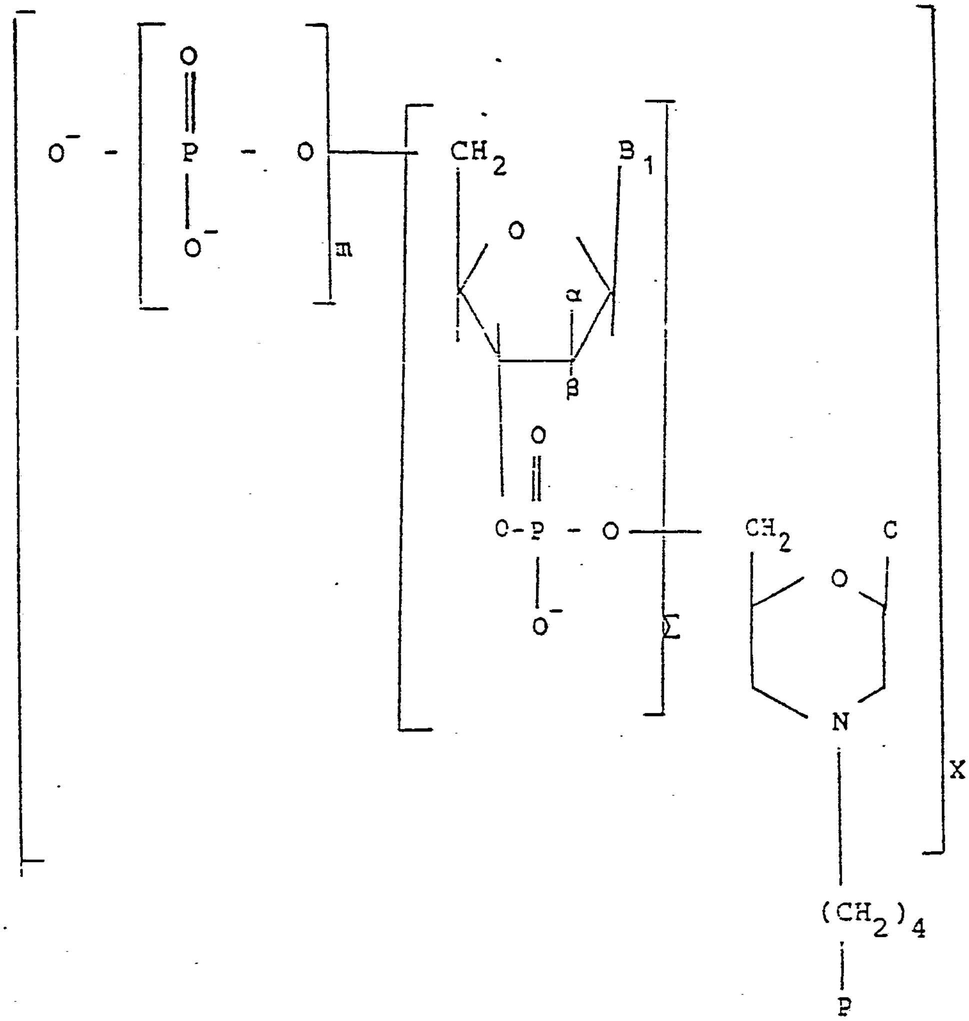

dans laquelle X, Σ, m, α, β, $B_1$ et P ont les significations indiquées ci-dessus et C représente la cytidine.

Un autre groupe avantageux de conjugués de l'invention est constitué par ceux de formule VI, VIbis, VIter, VII, VIII, IX, dans lesquels $B_2$ représente la cytidine.

Un autre groupe avantageux de conjugués de l'invention est constitué par ceux de formule XI suivante :

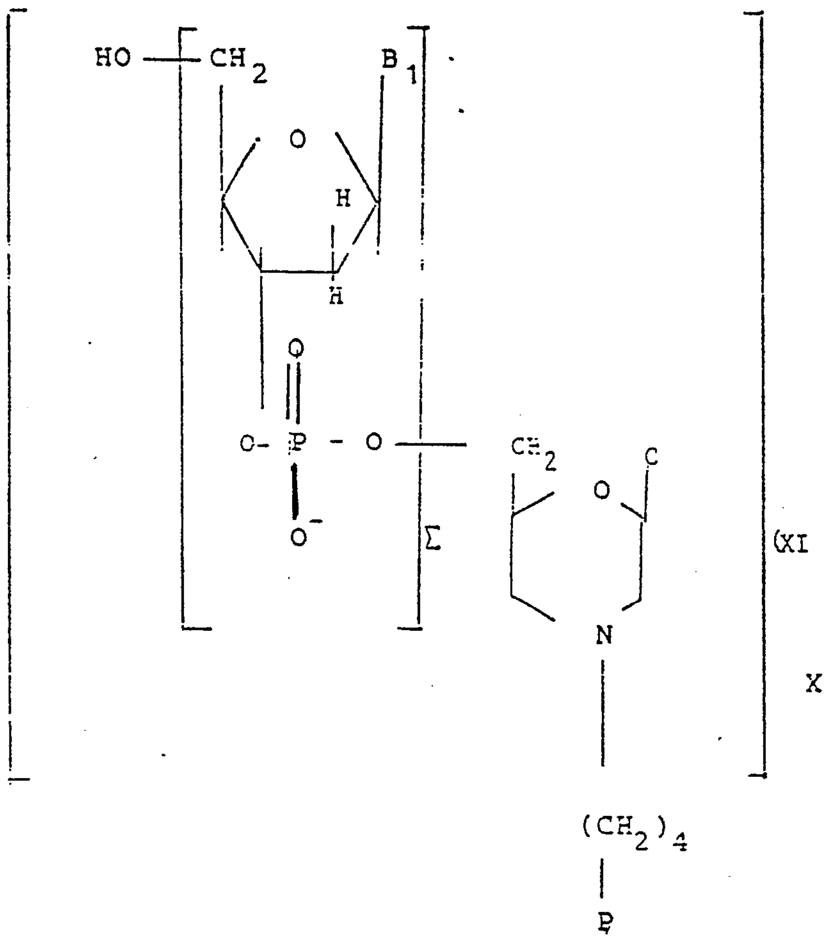

(XI )

dans laquelle $B_1$ et P ont la signification indiquée ci-dessus, C représente la cytidine, Σ vaut 12 à 15 et X vaut 1 à 5, de préférence 1.

Un autre groupe avantageux de conjugués de l'invention est constitué par ceux de formule V, VI, VIbis, VIter, VII, VIII, IX, X, XI, dans lesquels P a un poids moléculaire d'environ 5 000 à environ 40 000, notamment d'environ 10 000 à environ 20 000, et avantageusement d'environ 15 000.

Des conjugués particulièrement avantageux sont ceux répondant aux formules suivantes :

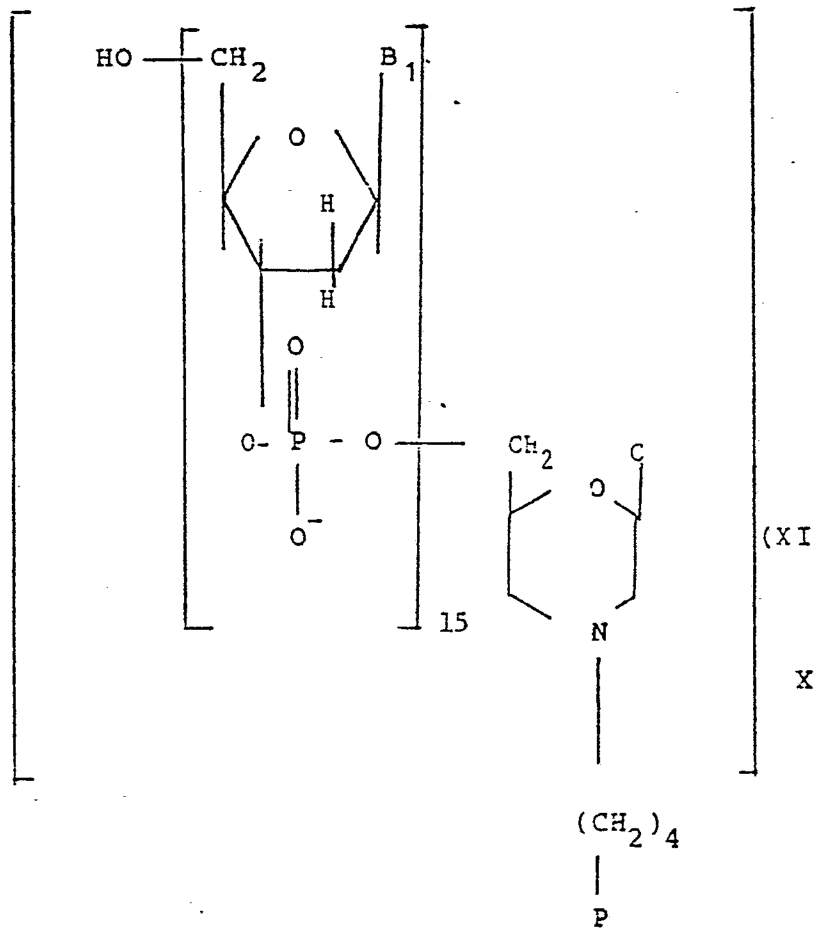

(XII)

dans laquelle
- P représente la poly (L) lysine de poids moléculaire d'environ 15 000,
- C représente la cytidine,
- Σ vaut 15, et
- l'enchaînement des bases $B_1$ est le suivant

3' TGTCATTAGTTTTAC 5'

et de formule XIII suivante :

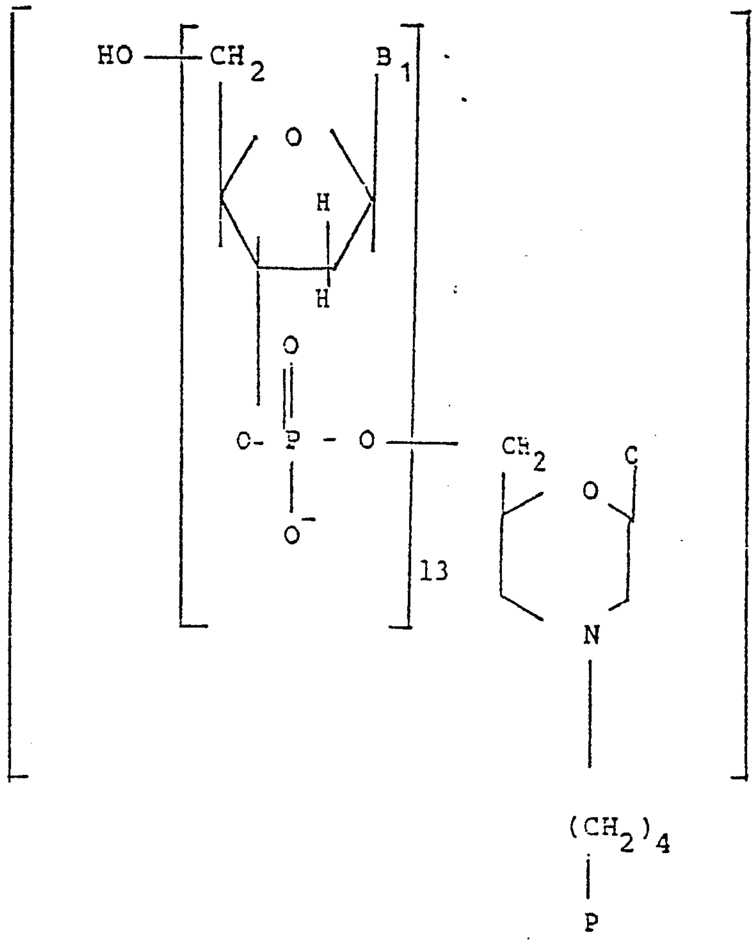

(XIII)

X

dans laquelle
- P représente la poly (L) lysine de poids moléculaire d'environ 15 000,
- C représente la cytidine,
-Σ vaut 13 et
- l'enchaînement des bases $B_1$ est le suivant

3' TTACACGGAGCAA 5'

En ce qui concerne le couplage de séquences oligonucléotidiques antisens courtes, on a constaté que couplage des séquences oligonucléotidiques antisens relativement courtes (7 à 15 nucléosides) à une séquence polypeptidique (comme par exemple la poly L-lysine) stabilise les premières vis-à-vis d'enzymes dégradatives (phosphodiesterase par exemple) et favorise également leur hybridation à la séquence oligonucléotidique cible.

L'invention concerne également les sels qui peuvent être obtenus par réaction des susdites séquences d'ARN ou d'ADN avec les bases appropriées, en particulier tels que les sels de sodium, sels d'alkyl ammonium, le radical alkyl ayant 1 à 5 atomes de carbone.

L'invention concerne également un procédé de préparation des conjugués de couplage.

Pour préparer les conjugués de couplage selon l'invention, on peut avoir recours au procédé décrit ci-après.

Ce procédé comprend
- la synthèse du conjugué de formule Ibis suivante

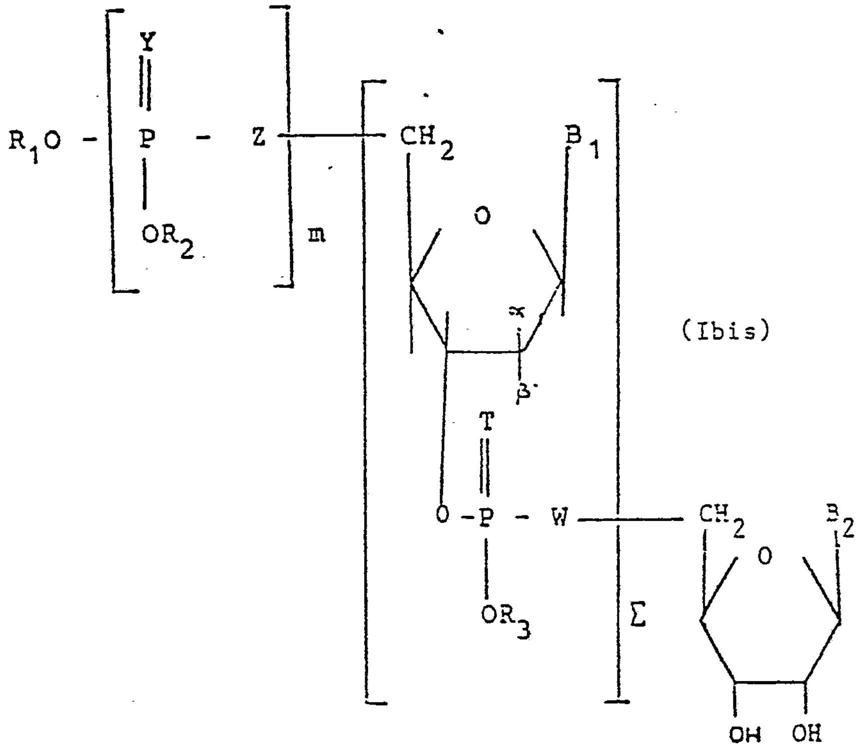

(Ibis)

dans laquelle $R_1$, $R_2$, $R_3$, Σ, m, $B_1$, $B_2$, Y, Z, T, W ont les significations indiquées ci-dessus,
- l'oxydation du conjugué de formule Ibis pour introduire deux fonctions aldéhyde sur les carbones en 2′ et 3′ de la dernière unité nucléosidique et obtenir le composé de formule Iter suivant :

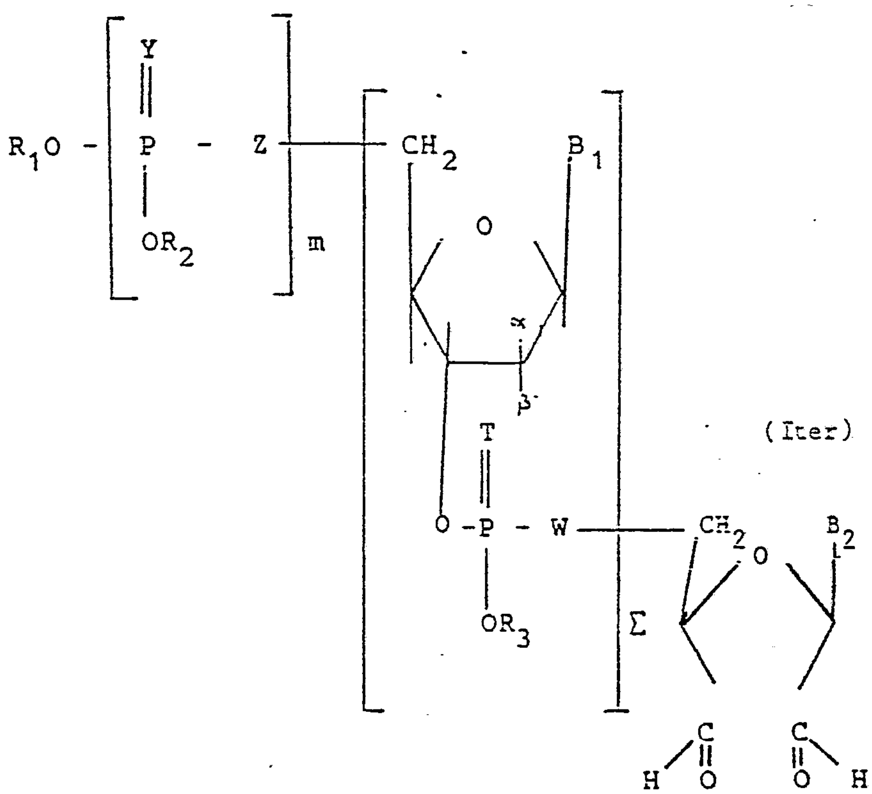

(Iter)

- l'alcoylation de façon réductive du conjugué de formule Iter par un polypeptide P-$NH_2$ dont l'enchaînement comprend au moins une séquence latérale dont l'extrémité est un groupe aminée, notamment $NH_2$, cette réaction ayant lieu entre les fonctions aldéhyde du conjugué (Iter) avec le groupe $NH_2$ du polypeptide pour obtenir le conjugué de formule (I)

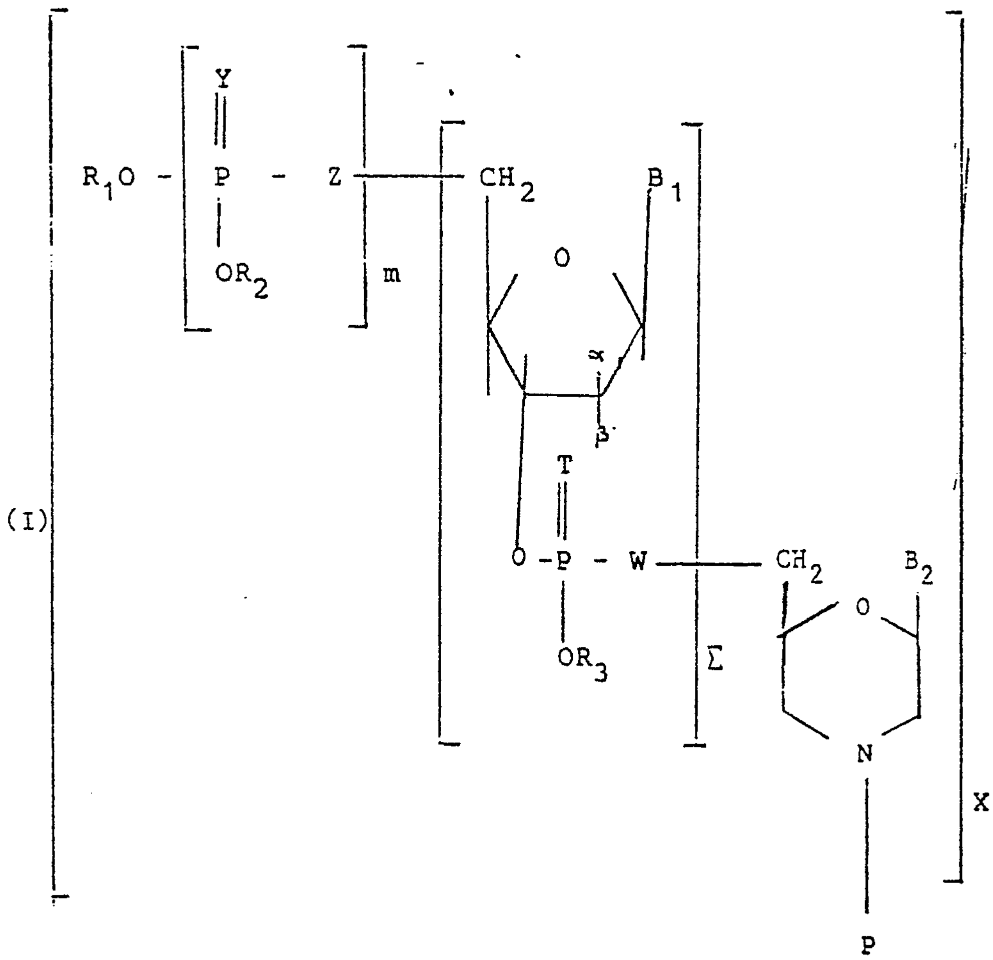

- le fractionnement entre les conjugués de couplage de formule I et les conjugués Iter et $P-NH_2$ non couplés, notamment par filtration moléculaire.

L'oxydation se fait notamment par le périodate, notamment périodate de sodium, dans des conditions de pH rigoureusement contrôlées.

L'expression "conditions de pH rigoureusement contrôlées " signifie le maintien du milieu réactionnel à pH 4, 5 à 0-4°C et dans l'obscurité.

L'alcoylation réductive du composé de formule (Iter) se fait par des méthodes classiques telles que celles décrites dans Khym J.C., Biochemistry, 1963, 2, 344-350 ; Gray C., Arch. Biochem. Biophys., 1974, 163, 426-428 ; Lee R.T. et Lee Y.C., Methods in Enzymol., 1982, 83, 289-295 ; Imai J., Johnson M., Torrence P., J. Biol. Chem., 1982, 21, 12 739-12 741.

On peut utiliser notamment le borohydrure de sodium, ou de préférence la cyanoborohydrure de sodium, pour effectuer l'alcoylation réductive du composé (Iter).

Le nombre X de séquences oligonucléotidiques par rapport à un polypeptide de P dépend des quantités respectives du composé (Iter) par rapport à la quantité de polypeptide utilisé.

En ce qui concerne la synthèse chimique des conjugués de formule Ibis, elle a lieu à partir de la synthèse des composés de formule Iquater suivante :

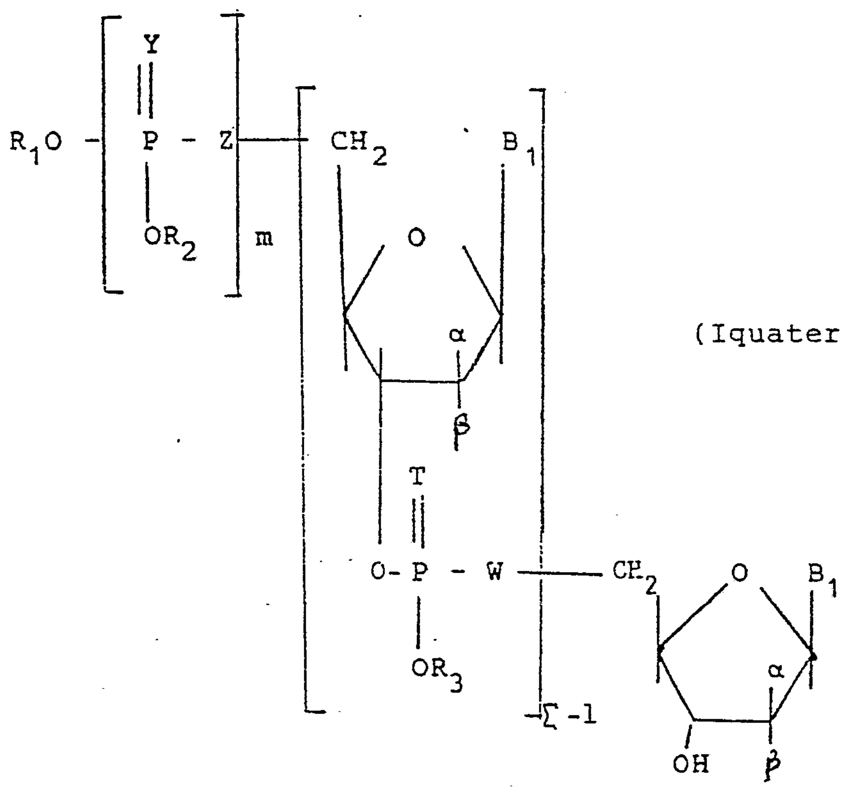

(Iquater)

dans laquelle $R_1$, $R_2$, $R_3$, $B_1$, m, Σ, Y, Z, T, W ont les significations indiquées ci-dessus, (α, β) représente (H, H), (H, OH) ou (OH, H).

Lorsque (α, β) représente (H, H) ou (H, OH), les composés Iquater correspondent respectivement à une séquence d'ADN ou d'ARN qui peuvent être synthétisés soit par voie chimique totale, notamment à l'aide d'un synthétiseur d'ADN ou d'ARN, soit par voie enzymatique.

En ce qui concerne la synthèse chimique, on peut se reporter au protocole décrit pour la synthèse d'ADN dans (Khorana et al., J. mol. Biol. 72, 209 (1972), J.E. Davies et H.G. Gassen, Angew. Chem. Int. Ed. Eng. 22, 13 (1983) ; K. Itakwa, J.J. Rossi et R.B. Wallace, Ann. rev. Biochem 53, 323 (1984) et pour la synthèse d'ARN au protocole décrit dans (T. Tonaka et al. Nucl. Acid. Res. 14, 6265 (1986) ; M. Sekine et T. Mata, J.A.C.S. 108, 4531 (1986).

En ce qui concerne la synthèse enzymatique des conjugués de formule Iquater, les techniques de production des séquences d'ADN ou d'ARN recombinants disponibles sont toutes potentiellement utilisables comme décrit par exemple pour l'ADN dans T. Maniatis, E.F. Fritsch et J. Sambrook Molecular Cloning, a laboratory manual (1982) et pour l'ARN dans P.A. Krieg et D.A. Melton Nucl. Ac. Res. 12, 7057 (1984).

Les composés de formule Ibis dans lesquels (α, β) représente (H, OH) sont des séquences d'ARN et par conséquent peuvent être synthétisés directement.

La préparation des composés de formule Ibis

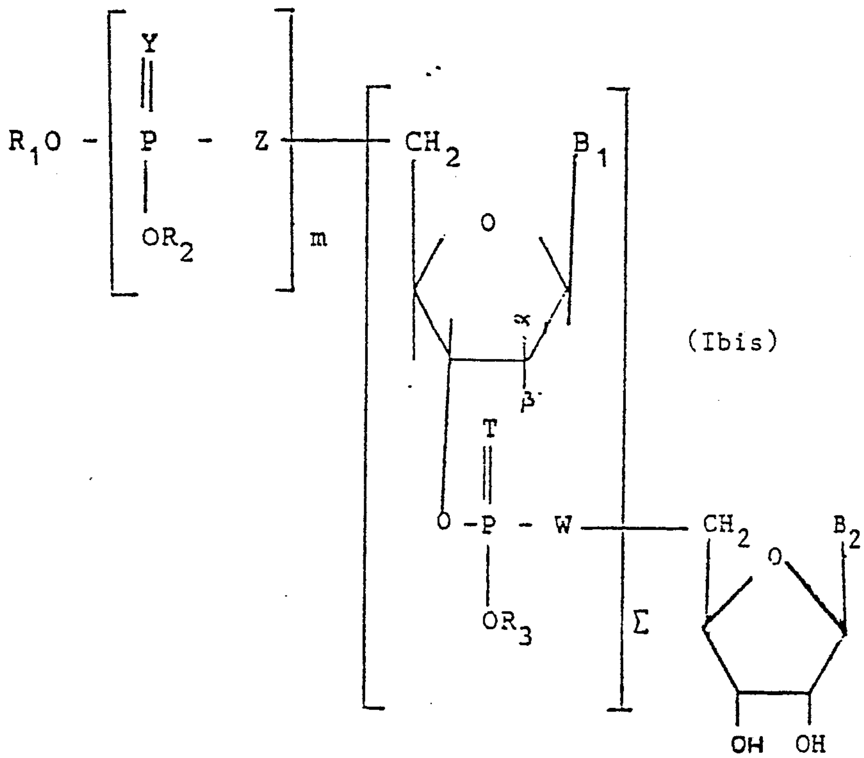

(Ibis)

dans laquelle $R_1$, $R_2$, $R_3$, Σ, m, $B_1$, $B_2$, Y, Z, T, W ont les significations indiquées ci-dessus et (α, β) représente (H, H) ou (OH, H), peut être obtenue à partir du composé de formule Iquater

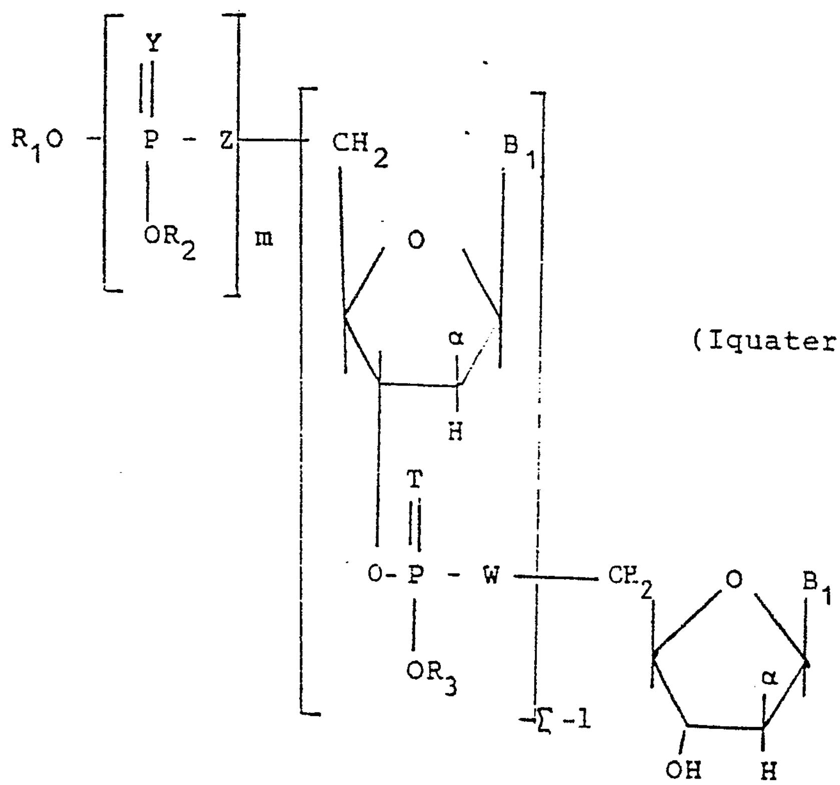

(Iquater)

dans laquelle $R_1$, $R_2$, $R_3$, $B_1$, m, Σ, Y, Z, T, W ont les significations indiquées précédemment, et α représente H ou OH, sur lequel on fait réagir le composé de formule

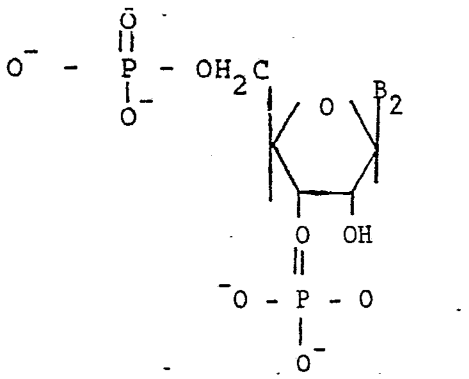

(Iquinquies)

dans laquelle $B_2$ représente A, T, C, G ou U et avantageusement C, en présence de RNA ligase de $T_4$ ($T_4$ ligase) pour obtenir le composé de formule Isexies suivante :

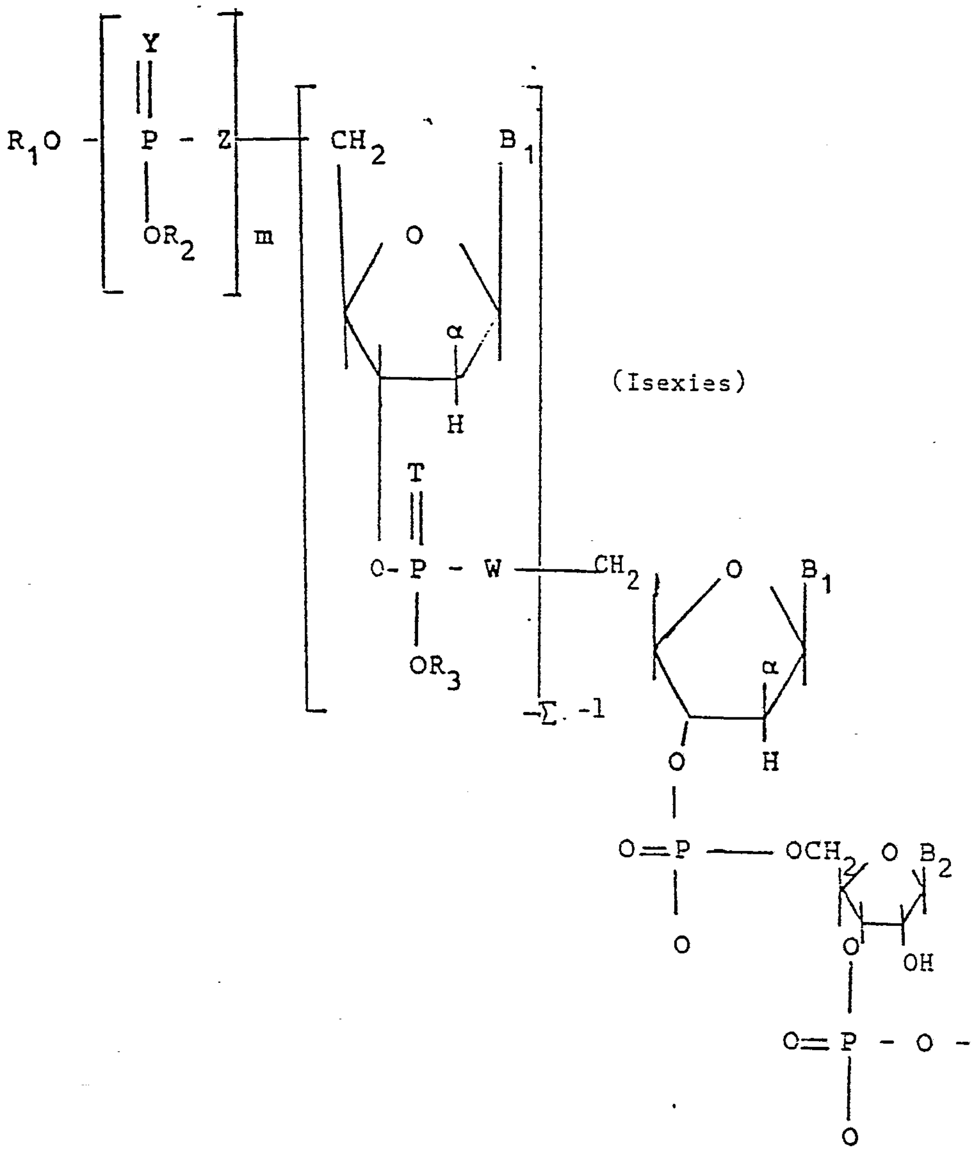

(Isexies)

- puis on effectue une filtration sur gel pour séparer le composé Isexies des composés Iquater et Iquinquies n'ayant pas réagi ;
- puis on fait agir la phosphatase alcaline bactérienne pour donner le composé de formule Ibis ci-dessus définie ou de formule (Ibis)',

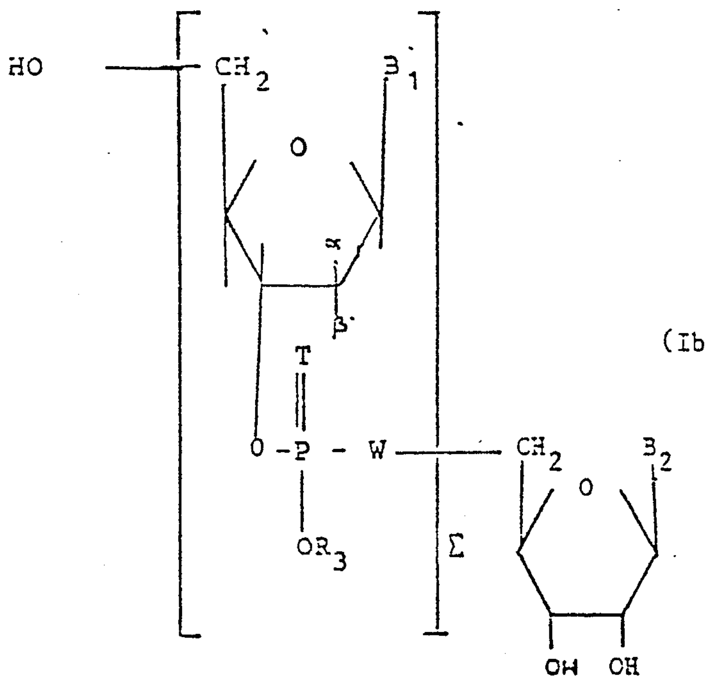

(Ibis)'

lorsque le groupe

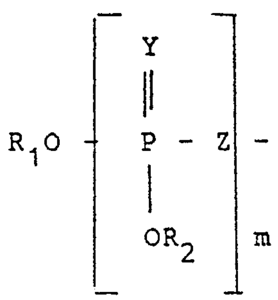

représente le groupe

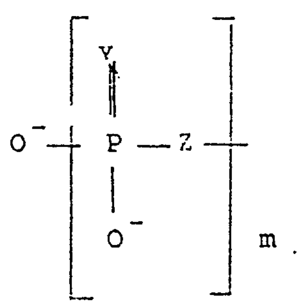

m, lequel a été également éliminé par la phosphatase alcaline bactérienne lorsque les oxygènes ne sont pas protégés par des radicaux $R_1$ et $R_2$.

En ce qui concerne le couplage à la $T_4$ ligase, on peut se reporter aux références suivantes : D.M. Hinton and R.I. Gumport (1979) Nucl. Ac. Res. 7, 453-465, C.A. Brennan, A.E. Manthey et R.I. Gumport (1983) dans Methods in Enzymology vol. 100, 38-52.

Avantageusement, la synthèse chimique des composés de formule Ibis pour lesquels $R_1$, $R_2$, $R_3$, $\Sigma$, m, $B_1$, $B_2$, Y, Z, T, W ont les significations indiquées ci-dessus, et ($\alpha$, $\beta$) représente (H, H), (H, OH) ou (OH, H), est réalisée selon une méthode en phase solide utilisant un support ribosylé sur lequel sont successivement additionnés des unités nucléotidiques. Cette méthode en phase solide est notamment celle ci-après désignée par "méthode phosphoramidite" et qui est décrite dans Matteucci M.D., Caruthers M.H., Journal Am. Chem. Soc. 103, 3185 (1981). Cette méthode est également utilisable pour la synthèse des composés de formule Iquater précédemment cités. Le support ribosylé sus-mentionné est une résine, avantageusement des billes

de verre à porosité contrôlée (également connue sous le nom de "Controlled Pore Glass resine" ou résine CPG), porteuse d'un ribonucléoside de base quelconque A C G T ou U, ou de dérivé purique ou pyrimidique de ces bases, choisi notamment parmi les suivants :

- xanthine (2,6-dihydroxypurine),
- hypoxanthine (6-hydroxypurine),
- purine ((7-H-imidazo[4,5-d]pyrimidine),
- pyrimidine (1,3-diazine),
- adénine-1N-oxyde,
- cytosine-5-carboxylique acide,
- guanine-3N-oxyde,
- 2-thiouracile,
- 4-thiouracile,
- 5-carboxy-2-thiouracile,
- 6-carboxymethyluracile,
- uracile-5-carboxylique acide,
- uracile-6-carboxylique acide,
- 8-bromo adénine,
- 5-bromo cytosine,
- 8-bromo guanine,
- 6-bromo purine.

Les conditions dans lesquelles de telles résines ribosylées sont préparées et utilisées pour la synthèse de séquences oligonucléotidiques sont précisées dans les exemples qui suivent.

Les conjugués de couplage de l'invention peuvent être avantageusement utilisés comme réactifs biologiques, notamment comme sondes de détection.

L'invention concerne également un procédé in vitro d'inhibition de l'expression d'un gène, notamment dans un milieu de culture cellulaire, à l'aide d'un au moins des conjugués de couplage ci-dessus envisagé.

Les exemples ci-après sont donnés à titre illustratif sans être limitatifs.

I- Synthèse des oligonucléotides de formule XII et XIII :

1) Matériaux et méthodes

Les milieux et sérum de veau foétal pour la culture cellulaire proviennent d'Eurobio (Paris). La poly (L-lysine) (M = 14 000), la phosphatase alcaline bactérienne (E.C. 3.1.3.1., - type III-r), la myokinase (E.C. 2.7.4.3., - type V), la spermine et la cyanoborohydure de sodium proviennent de Sigma.

Les supports de gel filtration sont fournis par Pharmacia Fine Chemicals (milieu Sephadex G-50) ou Bio-rad (Bio-Gel P6DG). La RNA ligase de $T_4$ (exemple de contamination par des RNases et/ou des DNases - E.C. 6.5.1.3.) et les composés de formule

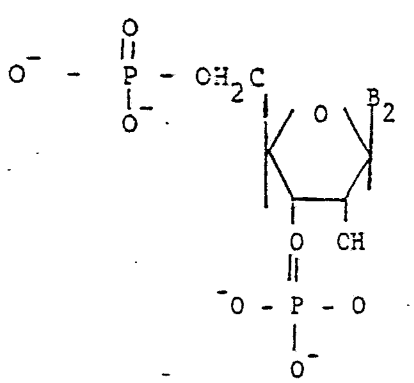

(Iquinquies)

appelé ci-après pCp non marqué proviennent de Pharmacia. La créatine kinase (E.C. 2.7.3.2.), la phosphocréatine, Hepes, le dithiotreitol et l'ATP proviennent de Boehringer.

La 5'-[$^{32}$P]-pCp (111 TBq/mmole) et la [$^{35}$S] méthionine (22-30 TBq/mmole) proviennent d'Amersham et les protéines de poids moléculaire standard proviennent de la société NEN.

2) Cellules et virus

Les cellules L929 sont cultivées dans un milieu essentiel minimum (MEM) additionné de 5 % (v/v) de sérum de veau foetal, 3 g/l de bouillon de phosphate bactotryptose, 3,4 g/l de glucose, 60 IU/ml de pénicilline et 50 mg/ml de streptomycine.

La souche Indiana du VSV ou le virus encéphalomyocarditis (EMCV) sont cultivés sur des monocouches de cellules L929 et titrés par une méthode de dilution limite (MILHAUD, P.G., SILHOL, M., FAURE, T. and

MILHAUD, X. (1983). Ann. Virol. (Inst. Pasteur) 134E, 405-416).

3) Synthèses des oligodéoxyribonucléotides

A. synthèse des oligodéoxyribonucléotides-pCp (composés de formule Isexies)

A.1. synthèse des composés de formule Iquater

Les oligodéoxyribonucléotides du type Iquater sont synthétisés en utilisant un synthétiseur d'ADN commercialisé par la Société Biosearch (modèle Sam One) en utilisant la méthode au phosphotriester (KHORANA, H.G., AGARWARL, K.L., BUECHI, H., CARUTHERS, M.H., GUPTA, N.K., KLEPPE, K., KUMAR, A., OHTSUKA, E., RAJ BHARDARY, U.L., VAN DE SANDE, J.H., SCARAMELLA, U., TERAO, T., WEBER, H. and YAMADA, T.. (1972). J. Mol. Biol. 72, 209-217). Les indications d'utilisation du fabricant sont observées sans modifications. Les oligonucléotides déprotégés sont purifiés sur une colonne de Séphadex G-50, équilibrée et éluée avec du tampon bicarbonate triethylamine 10mM pH 7.5 (TEAB), et on récupère un produit de haut poids moléculaire correspondant aux oligodéoxyribonucléotides. Le tampon TEAB est éliminé par évaporation et la pureté de cette fraction est testée par un procédé d'électrophorèse sur gel de polyacrylamide (PAGE) (MANIATIS, T., JEFFREY, A. and KLEID, D.G. (1975). Proc. Natl. Acad. 72, 1184-1189). Si nécessaire, les oligonucléotides sont purifiés par le procédé PAGE dans des conditions similaires à celles de l'analyse.

A.2. Addition de pCp aux composés de formule Iquater

Les conditions adaptées sont celles des publications suivantes :

- HINTON, D.M. and GUMPORT, R.I. (1979). Nucleic Acid. res. 7, 453-465 ;
- BRENNAN, C.A., MANTHEY, A.E. and GUMPORT, R.I. (1983). Methods in Enzymology, 100, 38-52.

L'oligodéoxyribonucléotide (80 nmoles) est lyophilisé avec 320 nmoles de composé de formule

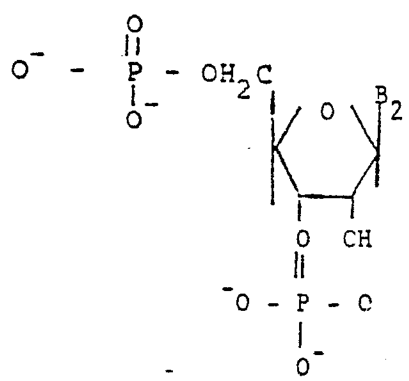

(Iquinquies)

dans lequel $B_2$ représente A, T, C, G ou U, et avantageusement C (pour la synthèse des composés XII et XIII), désigné ci-après par pCp, 1,85 MBq de 5'[$32_p$]-pCp, 80 nmoles de spermine, 1,8 µmoles de créatine phosphate et 4,5 nmoles d'ATP.

Le résidu est redissous dans 30 µl de tampon de ligation (acide <4-<2 hydroxyéthyle)- pipérazine éthane sulfonique) (Hepes), 50mM, pH 7.9 ; Mn $Cl_2$, 10nM ; (dithiothreitol) (DTT), 20mM, et on ajoute 1,7 U de créatine kinase et 1,7 U de myokinase dans 5 µl de tampon de ligation à 30 % (v/v) de glycérol et on initie la réaction par l'addition de la RNA ligase de $T_4$ (10-15 µl à une concentration finale de 23 µM).

On laisse incuber le mélange réactionnel à 18° C pendant 6 jours. Le pCp non lié et les sels sont éliminés par chromatographie sur une colonne de Biogel P6-DG équilibrée et développée avec 20mM de TEAB. On mesure la radioactivité à l'absorbance de 260 nm de chacune des fractions. Les fractions contenant l'oligodéoxyribonucléotide-pCp sont rassemblées et évaporées à sec.

B. synthèse directe des oligonucléotides de formule Ibis par la méthode phosphoramidite

Les oligonucléotides du type Ibis, encore appelés dans ce qui suit oligodéoxyribo-N, N représentant un ribonucléotide symbolisé par rX, X représentant A C T G ou U, ou des dérivés de ces bases tels que définis ci-dessus (avantageusement C dans le cas de la synthèse des composés XII et XIII), sont synthétisés en utilisant un synthétiseur d'ADN selon la méthode phosphoramidite (Matteucci M.D., Caruthers M.H., Journal Am. Chem. Soc. 103, 3185 (1981)). Cette méthode utilise une résine CPG ribosylée dont la préparation est, par exemple, réalisée de la manière suivante :

B.1. préparation du composé de formule :

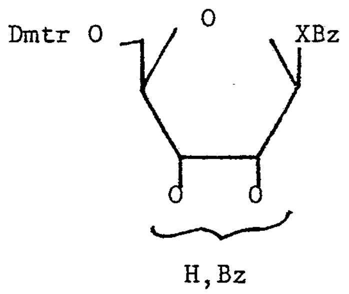

dans laquelle . Dmtr représente le groupe diméthoxytrityle,

. XBz représente une base A U C G T, ou un dérivé de ces bases, dont les groupes réactionnels amines ou hydroxyles portent des groupes benzoyle (Bz),

. la représentation

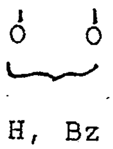

signifie que l'un des deux atomes d'oxygène en 2' ou 3' porte un groupe benzoyle (Bz), tandis que l'autre porte un hydrogène.

Cette formule correspondant au ribonucléoside-X diméthoxytritylé benzoylé peut encore être schématisée de la façon suivante

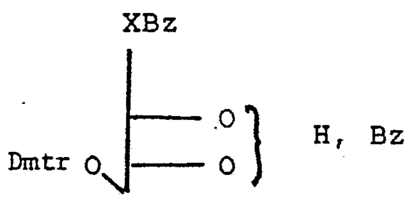

La préparation de ce composé est décrite dans Kempe, Chow, Sundquist, Nardi, Paulson, and Peterson (1982) N.A.R. 10(21), 6695.

B.2. préparation du composé de formule

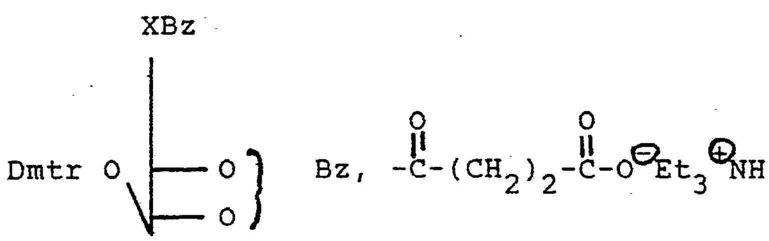

La préparation de ce composé est décrite dans Chow, Kempe, and Palm (1981) N.A.R., 9, 2807.

B.3. préparation de l'ester actif

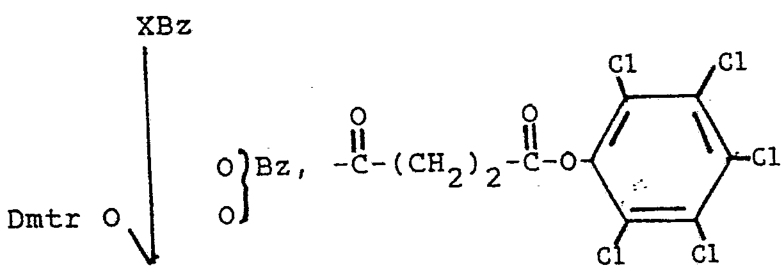

Elle est décrite dans Miyoshi K., Arentzen R., H-uang T., and Itakura K. (1980), N.A.R., 8, 5507.

B.4. préparation du support :

La fixation du produit obtenu en B.3. sur la résine CPG activée (disponible commercialement) est effectuée selon Gough R., Brunden M.J., Gilham P.T., (1981) Tetrahedron Lett., 22, 4177.

Les déoxyribonucléotides sont ensuite successivement additionnés sur ce support, avantageusement au moyen d'un synthétiseur automatique d'oligonucléotides selon la méthode décrite dans l'article de Matteucci et Caruthers sus-mentionné, ou selon une procédure dérivée de cette dernière, ou toute autre méthode appropriée. La déprotection de l'oligodéoxynucléotide est réalisée dans $NH_3$, puis il est précipité par l'éthanol (2,5 volumes pour 1 volume de solution contenant l'oligonucléotide), le culot est séché, et la pureté de cette fraction est testée par un procédé d'électrophorèse sur gel de polyacrylamide (PAGE)(MANIATIS, T., JEFFREY, A. and KLEID, D.G. (1975). Proc. Natl. Acad. 72, 1184-1189). Si nécessaire, les oligonucléotides sont purifiés par le procédé PAGE dans des conditions similaires à celles de l'analyse.

4) Conjugaison des oligodéoxyribonucléotides-pCp obtenus en A.3. à la poly (L-lysine)

Les conditions sont adaptées à partir de la méthode décrite notamment dans (BAYARD, B., BISBAL, C. and LEBLEU, B., (1986). Biochemistry 25, 3730-3736).

On solubilise l'oligodéoxyribonucléotide-pCp sec dans 50 µl de TEAB 10mM et on incube pendant 1 h à 37°C avec 1 unité de phosphatase alcaline bactérienne. On filtre l'oligodéoxyribonucléotide déphosphorylé sur une colonne Biogel P6-DG dans du TEAB 10mM et les fractions contenant l'oligodéoxyribonucléotide sont lyophilisées. Le résidu est solubilisé dans 100 µl de métapériodate de sodium (1 µmole dans un tampon acétate sodium 0,1M pH 4.75) et on incube le mélange réactionnel pendant 2 h à 0-4°C dans l'obscurité. On ajoute ensuite 100 µl de poly (L-lysine) (80 nmoles dans un tampon phosphate 0,2M, pH 8.0) et 100 µl de cyanoborohydrure de sodium (10 µmoles dans un tampon phosphate 0,2M, pH 8.0) et on laisse incuber pendant 2 h à la température ambiante. L'échantillon est ensuite déposé sur une colonne de Séphadex G-50 équilibrée et éluée avec du tampon acétate d'ammonium 0,1 M, pH 4.75. On teste chaque fraction relativement à son contenu en oligonucléotides-poly(L-lysine) en utilisant la méthode de Lowry (LOWRY, O.H., ROSEBROUGH, N.S., FARR, A.L. and RANDALL, R.I. (1951). J. Biol. Chem. 193, 265-275), l'absorption à 260 nm, et on mesure la radioactivité.

5) Conjugaison des oligodéoxyribo-N obtenus en B.3.à la poly (L-lysine)

L'oligodéoxyribo-N (N=rC dans le cas des composés XII et XIII) synthétisé en 3)B. est solubilisé dans 100 µl de métapériodate de sodium (1 µmole dans un tampon acétate sodium 0,1M pH 4.75) et on incube le mélange réactionnel pendant 2 h à 0-4°C dans l'obscurité. On ajoute ensuite 100 µl de poly (L-lysine) (80 nmoles dans un tampon phosphate 0,2M, pH 8.0) et 100 µl de cyanoborohydrure de sodium (10 µmoles dans un tampon borate 0,2M, pH 8.0) et on laisse incuber pendant 2 h à la température ambiante. L'échantillon est ensuite déposé sur une colonne de Séphadex G-50 équilibrée et éluée avec du tampon acétate d'ammonium 0,02 M, NaCl 0,5 M, pH 6. On teste chaque fraction relativement à son contenu en oligonucléotides-poly(L-lysine) en utilisant la méthode de Lowry (LOWRY, O.H., ROSEBROUGH, N.S., FARR, A.L. and RANDALL, R.I. (1951). J. Biol. Chem. 193, 265-275), l'absorption à 260 nm.

6) Test de synthèse protéique

On lave les cellules L929 (3 à 5 x $10^5$ cellules) deux fois avec du milieu essentiel minimum (MEM) libre de méthionine et on les marque pendant 45 min à 37°C avec 185 kBq de méthionine [$^{35}$S] dans 500 )l de milieu essentiel minimum, carencé en méthionine. Les protéines sont extraites et analysées par un procédé de gradient SDS-PAGE (7.5 - 15 % ; w/v) comme décrit précédemment (BAYARD, B., BISBAL, C. and LEBLEU, B., (1986). Biochemistry 25, 3730-3736). La distribution de protéines dans les gels est analysée par fluorographie (LASKEY, R. and HILLS, A. (1975). Eur. J. Biochem. 56, 335-341) et quantifiée avec un densitomètre de marque Vernon sur les autoradiographies.

II- RESULTATS

Conjugaison d'oligodéoxyribonucléotides et de poly (L-lysine)

Les oligoribonucléotides peuvent être liés de façon covalente à la poly (L-lysine) par un noyau N-morpholino après oxydation périodique de leur résidu ribose 3'-terminal (KHYM, J.C. (1963). Biochemistry 2, 344-350 ; RAYFORD, R., ANTHONY, D.D.Jr, O'NEIL, R.E. Jr and MERRICK, W.C. (1985). J. Biol. Chem. 260, 15708-15713). On additionne un motif ribose oxydable 3'-terminal afin d'utiliser cette méthode avec les oligodéoxyribonucléotides synthétiques. Cette opération peut être effectuée avec de la RNA ligase de $T_4$ qui forme une liaison phosphodiester 3', 5' entre l'hydroxyl-3' d'un oligonucléotide accepteur (c'est-à-dire l'oligodéoxyribonucléotide synthétique) et un oligonucléotides portant un phosphate-5' (donneur) tel qu'un nucléotide 3'-5' bisphosphate (i.e. pCp) (BRENNAN, C.A., MANTHEY, A.E. and GUMPORT, R.I. (1983). Methods in Enzymology, 100, 38-52 ; UHLENBECK, O.C. and GUMPORT, R.I. in "The Enzymes" (P.G. Boyer, ed) 3rd Ed. vol. 15, p. 31. Academic Press, New York, 1982). On peut lier [$^{32}$P]pCp et des oligodéoxyribonucléotides à des rendements atteignant et dépassant 85 %.

En ce qui concerne la méthode phosphoramidite, elle présente l'avantage, d'obtenir directement des oligonucléotides synthétiques présentant un motif ribose oxydable 3' terminal, sans passer par une étape intermédiaire d'addition de composés de formule Iquinquiés (ou de pCp dans le cas des composés XII et XIII)

aux composés de formule Iquater. De plus, cette méthode phosphoramidite permet l'obtention d'oligonucléotides de taille supérieure à celle des composés obtenus par la méthode décrite dans le paragraphe 3)A.

On effectue ensuite l'oxydation des résidus 3'-terminaux de cytidyle et on effectue ensuite leur couplage aux groupes Σ-amino de la poly (L-lysine) (BAYARD B., BISBAL C., et LEBLEU B. (1986) Biochemistry, 25, 3730-3736)

On obtient de bons résultats en couplant 1 à 15, avantageusement de l'ordre de 1 à 3, oligodéoxyribonucléotides à de la poly (L-lysine) de poids moléculaire 14 KDa.

On rappellera que le génome du virus de la stomatite vésiculaire (VSV) est consistitué d'un simple brin d'ARN (de polarité négative) d'environ 11 000 nucléotides de long, qui code pour 5 protéines : N, NS, M, G, et L (HUANG, A.S. and WAGNER R.R. (1966). J. Mol. Biol. 22, 381-384 ; REPIK, P. and BISHOP, H.L. (1973). J. Virol. 12, 969-983).

La protéine N recouvre la longueur entière de l'ARN du virion et joue des rôles dans la réplication du génome et éventuellement dans sa transcription (WAGNER, R.R., (1975). In "Comprehensive Virology" (H. Fraenkel-Conaat and R.R. Wagner, Ed.) pp 1-93, Plenum Publishing Corp. New York). Dans les études faites jusqu'à présent, le meilleur effet inhibiteur des oligonucléotides synthétiques anti ARN messager est obtenu avantageusement avec des oligomères complémentaires à la région 5' de la cible d'ARN messager, y compris ou non le codon d'initiation AUG (COLEMAN J., HIRASHIMA A., INOKUCHI Y., GREEN P.J., et INNOUYE (1985), Nature, 315, 601-603 ; IZANT J.G., et WEINTRAUB H. (1985) Science, 229, 345-352 ; MILLER P.S., AGRIS C.H., AURELIAN L., BLAKE K.R., MURAKAMI A., REDDY M.P, SPITZ S.A, et TS'O P.O.P (1985) Biochimie 67, 769-776).

On synthétise ainsi deux oligodéoxyribonucléotides. Le premier (oligodéoxyribonucléotide 1, figure 1) comporte 15 nucléotides et est complémentaire à la région terminale 5' de l'ARN messager codant pour la protéine N du VSV. Il peut hybrider le site d'initiation protégé du ribosome principal autour du codon AUG proche de l'extrémité 5'.

Le second oligodéoxyribonucléotide (oligodéoxyribonucléotide 2, figure 1) a une séquence interne de 13 nucléotides. Il est situé au milieu de la région codante de l'ARN messager (Gallione, C.J. Greene, J.R., Iverson, L.E. and Rose J.K. (1981), J. Virol 39, 529-535).

Ces oligodéoxyribonucléotides synthétiques sont liés de façon covalente à la poly (L-lysine) comme indiqué ci-dessus. Les rapports moléculaires en moyenne d'oligodéoxyribonucléotide à la poly (L-lysine) sont d'environ 0,5 et 0,6 respectivement. Les rendements sont d'environ 25 et 30 % respectivement.

Effet antiviral des conjugués oligodéoxyribonucléotide-poly (L-lysine)

On incube les conjugués complémentaires de la séquence terminale 5' et définis comme indiqué ci-dessus avec des cellules L929, 2 heures avant l'infection avec VSV, à une multiplicité d'infections de 1. Les titres en virus sont déterminés 12 heures plus tard par l'essai de dilution limite (MILHAUD, P.G., SILHOL, M., FAURE, T. and MILHAUD, X. (1983). Ann. Virol. (Inst. Pasteur) 134E, 405-416). Des expériences préliminaires indiquent que le conjugué est toujours efficace lorsque ajouté peu de temps (c'est-à-dire 2 heures) aprås l'infection avec VSV.

Les résultats relatifs à l'activité antivirale des conjugués d'oligonucléotides et de poly (L-lysine) figurent dans le tableau I ci-après.

TABLEAU I

ACTIVITE ANTIVIRALE DES CONJUGUES DE L'INVENTION

| Concentration en oligonucléotide nM | Séquence terminale 5' d'oligonucléotide liée à la poly(L-lysine) | | mélange de séquence terminale 5' et de poly(L-lysine) | séquence interne d'oligonucléotide liée à la poly(L-lysine) |
|---|---|---|---|---|
| | titre en VSV | titre en EMCV | titre en VSV | titre en VSV |
| 0 | Exp[t] 1 4.2 $10^8$ | 5.8 $10^7$ | 3.1 $10^8$ | 1.0 $10^8$ |
| 100 | 1.7 $10^8$(40%) | 3.1 $10^7$(53%) | 2.3 $10^8$(75%) | 7.8 $10^8$(78%) |
| 200 | 7.8 $10^6$ ( 2%) | 3.1 $10^7$(53%) | 4.2 $10^8$(135%) | 7.8 $10^7$(78%) |
| 400 | 3.0 $10^6$(0.7%) | 5.8 $10^7$(100%) | 2.3 $10^8$(75%) | 7.8 $10^7$(78%) |

On infecte par VSV ou EMCV des cellules L929 à la multiplicité d'infection de 1, deux heures après incubation avec le conjugué de l'invention ou la séquence d'oligodéoxyribonucléotide non conjuguée. Les titres en virus sont déterminés par l'essai de dilution limite 15 heures plus tard et exprimés en pfu/ml* ou en pourcentage de la valeur témoin (indiqué entre parenthèses).

* plate forming unit
= unité de formation de plage de lyse

On a indiqué dans la première colonne 1 le titre en VSV.

On observe que la réduction est dépendante de la dose, et que l'activité antivirale est détectable avec une

quantité aussi faible que 100 nM du conjugué de l'invention dans le milieu de culture et atteint plus de 99 % d'inhibition à 400 nM.

Dans un essai séparé (colonne 3 du tableau I), les cellules de L929 sont incubées avec un mélange de poly (L-lysine) et des séquences oligodéoxyribonucléotides 5′-terminal, avec aucune activité antivirale significative. Ce résultat montre que la liaison covalente de l'oligodéoxyribonucléotide à la poly (L-lysine) est nécessaire pour obtenir une activité biologique. Par ailleurs, aucun effet sur la multiplication virale n'a été observée lorsque les cellules sont incubées avec la poly (L-lysine) de masse moléculaire 14 000 à des doses allant jusqu'à 1 μM.

La colonne 2 du tableau I présente des résultats d'expériences différentes dans lesquelles les cellules de L929 sont incubées pendant 2 heure avec des conjugués de l'invention, avant infection avec le virus non apparenté EMCV. On détermine la quantité de virus 15 heures après l'infection. Quelle que soit la concentration du conjugué, aucun effet significatif sur la production d'EMCV n'est observé, à la différence de l'activité antivirale sur VSV. Ceci démontre l'aspect spécifique de l'action antivirale des séquences terminales 5′ conjuguées à la poly (L-lysine).

De plus, l'incubation des cellules avec un conjugué associant la poly (L-lysine) à un oligodéoxyribonucléotide complémentaire d'une séquence interne d'ARN messager de VSV codant pour la protéine N n'a pas d'effet significatif sur le titre de VSV mesuré 12 heures après l'infection. Les résultats figurent dans la colonne 4 du tableau 1. La localisation du site d'hybridation du conjugué de l'invention influence ainsi l'efficacité de l'inhibition des conjugués de l'invention. Ceci confirme les résultats des publications (IZANT, J.G. and WEINTRAUB, H. (1984). Cell 36, 1007-1015 ; IZANT, J.G. and WEINTRAUB, H. (1985). Science 229, 345-352 ; MILLER, P.S., AGRIS, C.H., AURELIAN, L., BLAKE, K.R., MURAKAMI, A., REDDY, M.P., SPITZ, S.A. and T'SO, P.O.P. (1985). Biochimie 67, 769-776) et montre que la région 5′ de l'ARN messager est une cible particulièrement appropriée relativement à l'inhibition de la traduction par les oligonucléotides antisens.

Effet des conjugués oligodéoxyribonucléotide-poly (L-lysine) sur la synthèse de protéine virale et cellulaire

On a examiné l'effet des conjugués sur la synthèse des protéines dans des cellules infectées et non infectées par VSV.

On incube des cellules L929 avec des concentrations croissantes de séquence terminal 5′ d'ARN messager conjuguée ou non à la poly (L-lysine), 2 heures avant l'infection avec VSV à une multiplicité d'infection de 1. Les cellules sont marquées avec de la méthionine [$^{35}$S] 5 heures après l'infection et on analyse les polypeptides acido-insolubles dans l'acide par le procédé d'électrophorèse SDS-PAGE. Le conjugué de l'invention conduit à une réduction (dépendante de la dose ) de la synthèse de la protéine N codée par VSV. On note que la synthèse de la protéine M codée par VSV est réduite de la même façon tandis que la synthèse des protéines cellulaires s'accroît de façon concomitante. Lorsque les cellules sont incubées avec un mélange de 200 pmoles d'oligodéoxyribonucléotide à séquence terminale 5′ et 400 pmoles de poly (L-lysine), c'est-à-dire dans le même rapport molaire et à la même concentration que lorsqu'on utilise le conjugué de l'invention, on n'observe aucun effet inhibiteur sur la synthèse des protéines virales, comparé au témoin. Ceci confirme la nécessité d'une liaison covalente entre la séquence oligodéoxyribonucléotide et un support polypeptidique pour l'activité biologique.

Dans un résultat séparé, la synthèse protéique est mesurée en l'absence ou en présence de 200 pmoles de conjugué de l'invention. Le conjugué n'a aucun effet sur la synthèse protéique cellulaire dans les cellules non infectées et l'augmentation de la synthèse des protéines cellulaires observée dans les cellules infectées par VSV résulte probablement d'un renversement partiel du blocage induit par VSV des synthèses protéiques de la cellule hôte.

On effectue l'analyse densitométrique des régions correspondant à la réduction de la synthèse de la protéine N codée par VSV, pour quantifier l'effet du conjugué de l'invention sur la synthèse protéique virale. La quantité de protéine virale N ou M est normalisée à un pic protéique cellulaire de 55 kDa. La figure 2 représente l'inhibition de la synthèse de protéine N et M par le conjugué de l'invention. La courbe repérée par des carrés noirs ■ correspond à la protéine N et celle repérée par des triangles noirs ▲ correspond à la protéine M. On a représenté en abscisse la concentration (en nM) de conjugué de l'invention et en ordonnée la quantité de protéine (N ou M) relative. Le rapport entre protéine virale et protéine cellulaire décroit de 25 à 0,1 pour la protéine N et de 13 à un niveau non mesurable pour la protéine M, parallèlement à une augmentation de concentration de conjugué de l'invention.

La figure 3 représente l'effet des conjugués de l'invention sur la synthèse protéique dans les cellules de L929 infectées par VSV et non infectées.

On incube les cellules L929 pendant 2 heures avec différentes concentrations des conjugués de l'invention avant l'infection (courbe représentée par des carrés noirs ■ ) avec VSV a une multiplicité d'infection de 1, ou en l'absence d'infection (courbe représentée par des ronds noirs ● ). Comme témoin, on incube des cellules L929 en présence de poly(L-lysine) avec ou sans 400 nM de séquence terminale 5′ d'oligodéoxyribonucléotide (rond blanc O ), les cellules sont marquées avec de la methionine [$^{35}$S] pendant 45 minutes, 6 heures après l'infection avec VSV. On mesure la radioactivité totale acido-insoluble.

On a indiqué en abcisse à la concentration en conjugué de l'invention (en nM), et en ordonnée l'incorporation de [$^{35}$S] methionine (en cpm x $10^{-3}$), (cpm : counts per minute = impulsion par minute).

Comme représenté sur la figure 3, la radioactivité total précipitable par l'acide demeure inchangée lorsqu'on incube les cellules non infectées avec des conjugués de l'invention. On observe une augmentation

dépendante de la dose dans la synthèse protéique lorsque les cellules sont traitées avec des concentrations croissantes du même conjugué de l'invention, mise en compétition avec VSV (multiplicité d'infection = 1) 2 heures plus tard, et marqué par de la méthionine [$^{35}S$] 6 heures après l'infection. Une fois encore un mélange d'oligodéoxyribonucléotide de 200 pmoles non conjugué et 400 pmoles de poly (L-lysine) ne modifie pas la synthèse protéique totale dans les cellules infectées par VSV.

Ce dernier ensemble de résultats confirme l'activité antivirale du conjugué, la nécessité de couplage pour observer une telle activité, la spécificité des conjugués de l'invention et l'absence de toxicité dans les doses utilisées dans les cellules L929.

L'ensemble des résultats démontrent que les conjugués de l'invention présentent une activité antivirale spécifique efficace vis-à-vis de VSV dans des cultures de cellules.

L'invention concerne également un réactif biologique comprenant comme élément actif l'un au moins des conjugués de couplage selon l'invention.

L'invention concerne également un procédé in vitro d'inhibition de l'expression d'un gène, notamment dans un milieu de culture cellulaire à l'aide d'un au moins des conjugués de couplage de l'invention.

## Revendications

1. Conjugués de couplage de formule I suivante

(I)

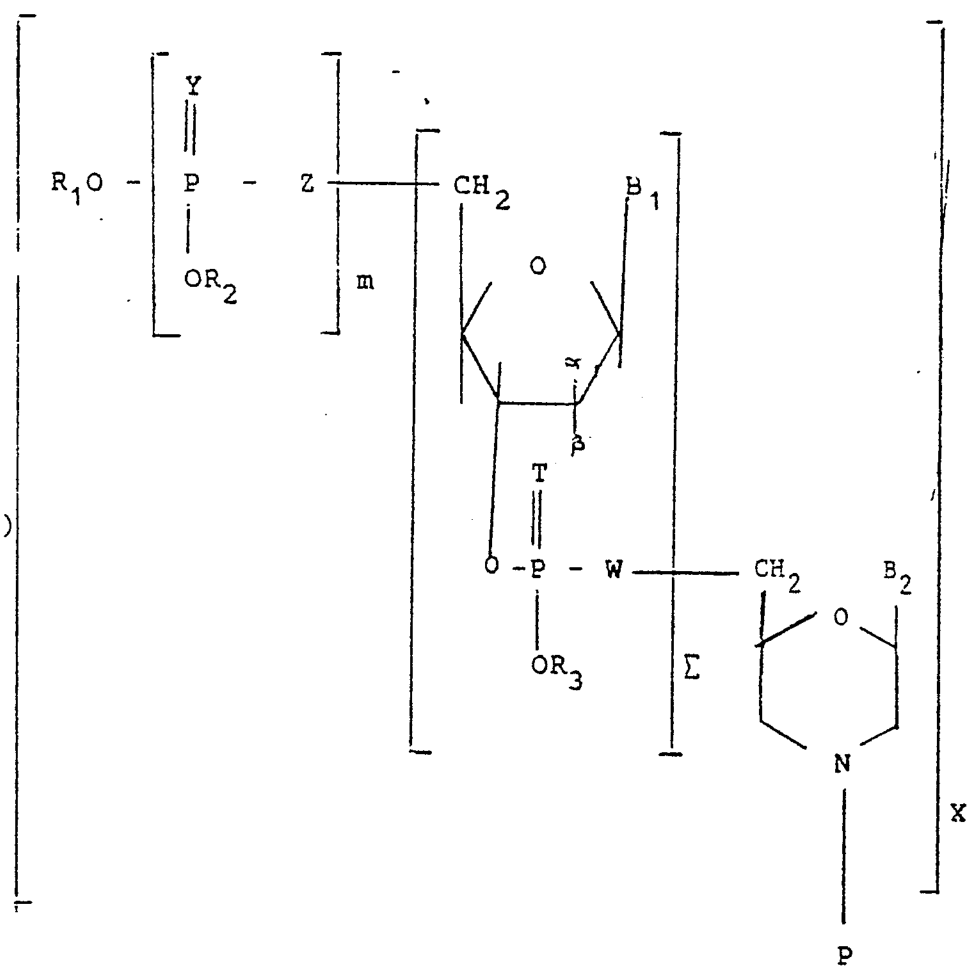

dans laquelle

- m est un nombre entier variant de 0 à 3, et représente de préférence 0 ou 1,
- $R_1$, $R_2$, $R_3$ sont identiques ou différents et représentent un alcoyle de 1 à 10 atomes de carbone, de préférence de 1 à 2 atomes de carbone, ou bien chacun des groupes $OR_1$, $OR_2$, $OR_3$ représente indépendamment l'un de l'autre 0-,
- Y et T, identiques ou différents, représentent O ou S,
- Z et W, identiques ou différents, représentent O, S $CH_2$, $CF_2$, ou NH,
- $B_1$ et $B_2$, identiques ou différents, représentent une base choisie parmi l'adénine (A), la guanine (G), la cytosine (C), la thymine (T) ou l'uracile (U),

- (α, β) représente (H,H), (H, OH) ou (OH, H),
- Σ est un nombre entier supérieur ou égal à 7, avantageusement compris de 7 à 30, de préférence de 7 à 20, et notamment de 12 à 15,
- X est un nombre entier compris de 1 à 5, et avantageusement vaut 1,
- P est un enchaînement polypeptidique comportant au moins 5 acides aminés, naturels ou synthétiques, et l'atome d'azote appartenant au cycle

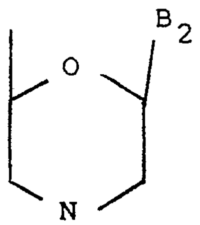

appartenant également à l'extrémité d'une séquence latérale du polypeptide P et /ou appartenant à un groupe $\underset{\underset{O}{\|}}{C}$-NH-N provenant d'une groupe COOH modifié par l'hydrazine.

2. Conjugué de couplage selon la revendication 1, caractérisé en ce que P est un enchaînement peptidique comportant au moins 5 acides aminés, et l'atome d'azote appartenant au cycle

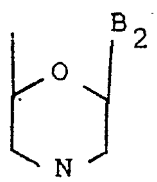

appartenant également à l'extrémité d'une séquence latérale du polypeptide P.

3. Conjugués de couplage selon la revendication 1, de formule II

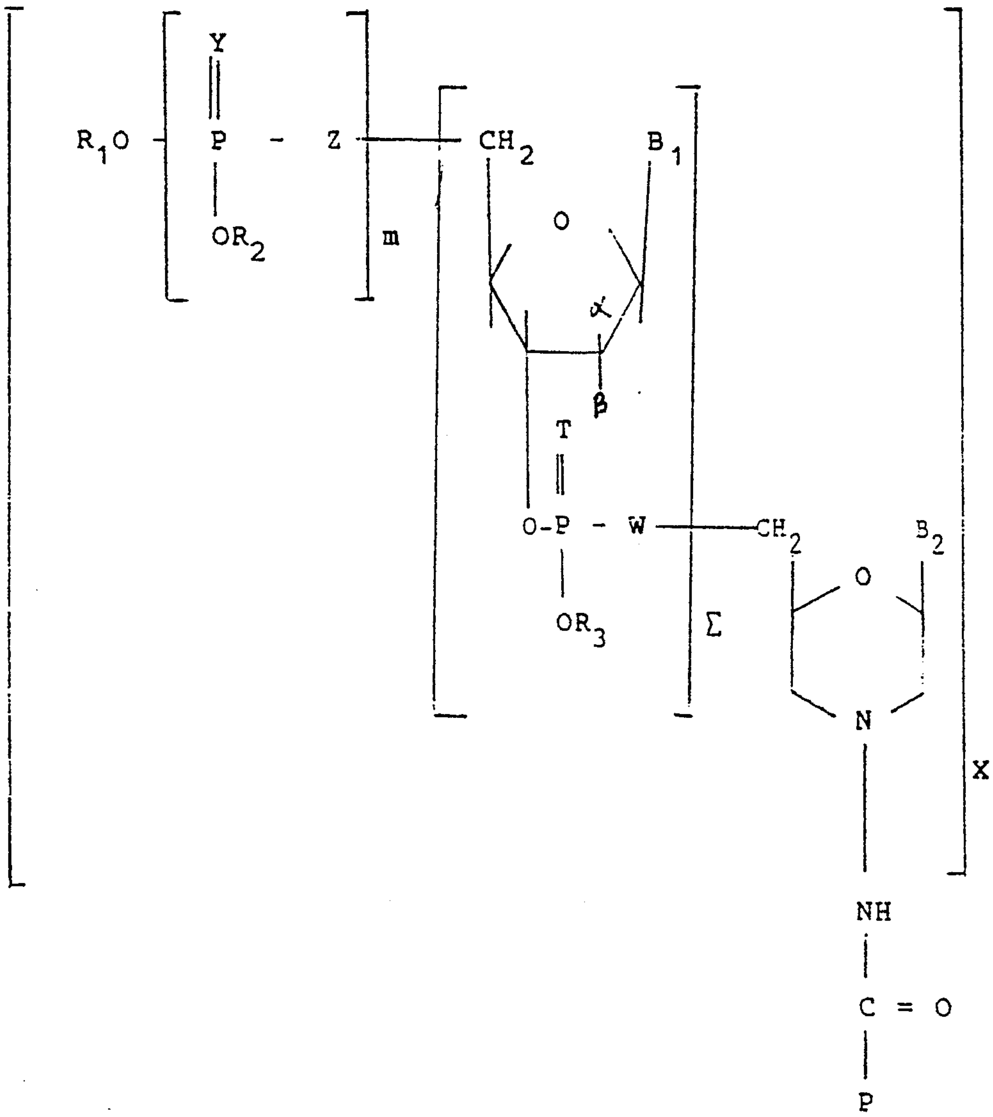

dans laquelle Y, Z, T, W, $\Sigma$, m, $\alpha$, $\beta$, X, $R_1$, $R_2$, $R_3$, $B_1$, $B_2$ ont les significations définies à la revendication 1, P′ représente un enchaînement polypeptidique, comportant au moins 5 acides aminés, et dans lequel le groupe

$\underset{\underset{O}{\|}}{C}$ - NH - N provient d'un groupe $\underset{\underset{O}{\|}}{C}$ - OH

terminal du polypeptide et/ou d'un groupe

- $\underset{\underset{O}{\|}}{C}$ - OH

libre d'un résidu aspartyle et/ou glutamyle.

4. Conjugués de couplage selon la revendication 1, de formule III

(III) 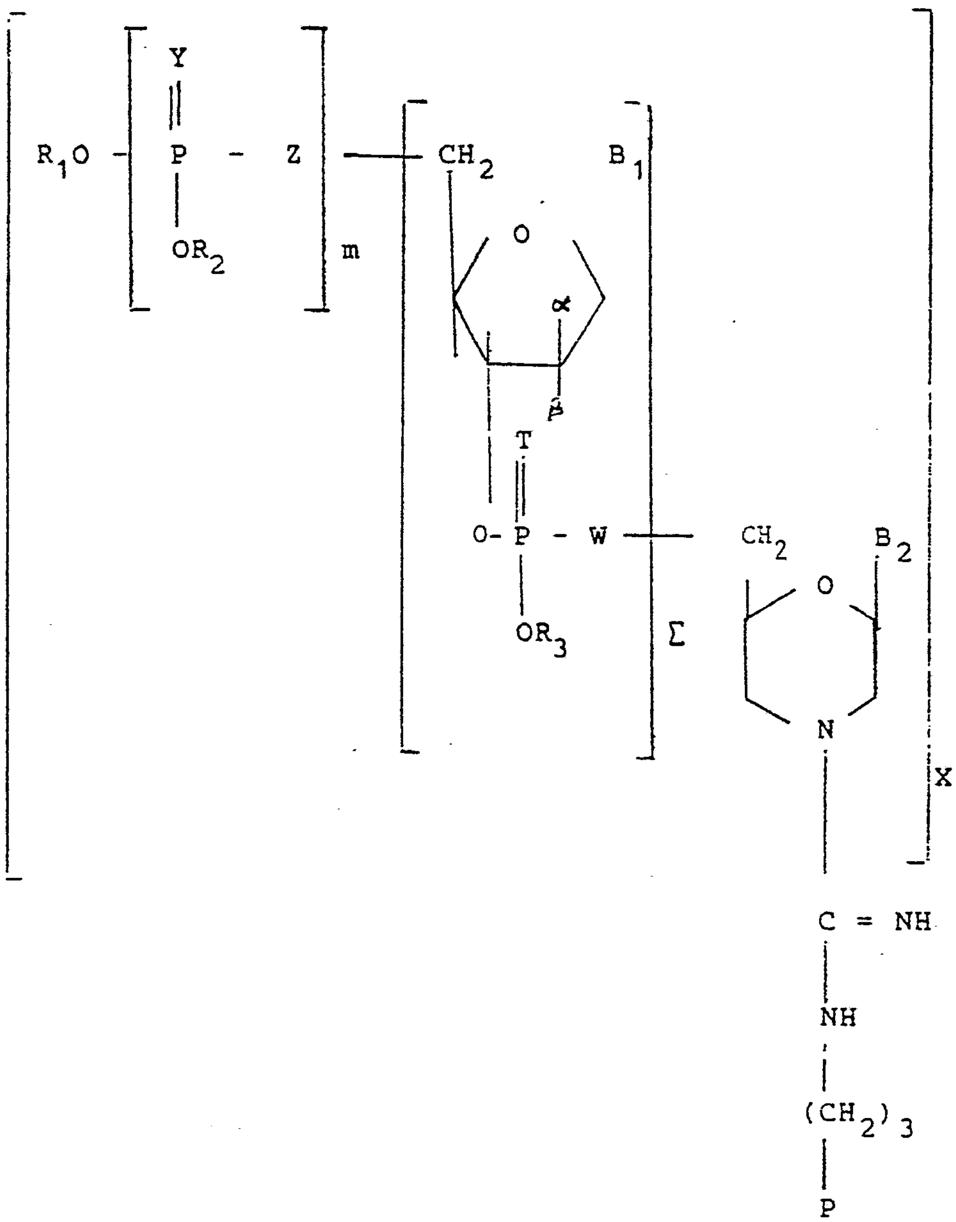

dans laquelle Y, Z, T, W, Σ, m, α, β, X, $R_1$, $R_2$, $R_3$, $B_1$, $B_2$ ont les significations définies à la revendication 1, P′ est un enchaînement peptidique comportant au moins 5 acides aminés et le groupe - $C(=NH)$ - NH - $(CH_2)_3$ - apparte-nant à l'extrémité de la séquence latérale d'un résidu arginyle entrant dans la constitution de l'enchaînement polypeptidique P′.

5. Conjugués de couplage selon la revendication 1, de formule IV

(IV) 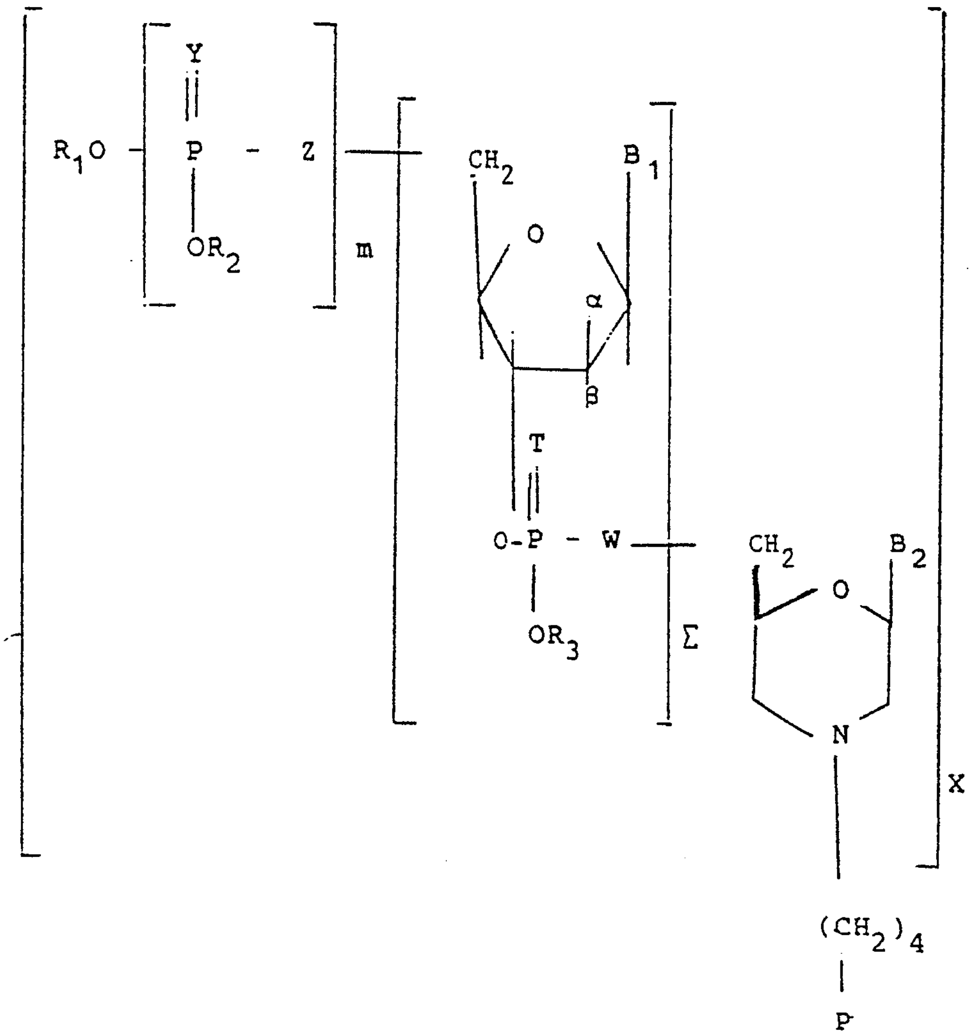

dans laquelle Y, Z, T, W, Σ, m, α, β, X, $R_1$, $R_2$, $R_3$, $B_1$, $B_2$ ont les significations définies à la revendication 1, et P' est un enchaînement peptidique comportant au moinss 5 acides aminés et dans laquelle le groupe $-(CH_2)_4$ - appartient à la séquence latérale d'un résidu lysyle entrant dans la constitution de l'enchaînement polypeptidique P.

6. Conjugués de couplage selon les revendications 1 à 5, dans lesquels l'un au moins des éléments Y ou Z est différent de l'oxygène.

7. Conjugués de couplage selon la revendication 5, de formule V suivante

(V) 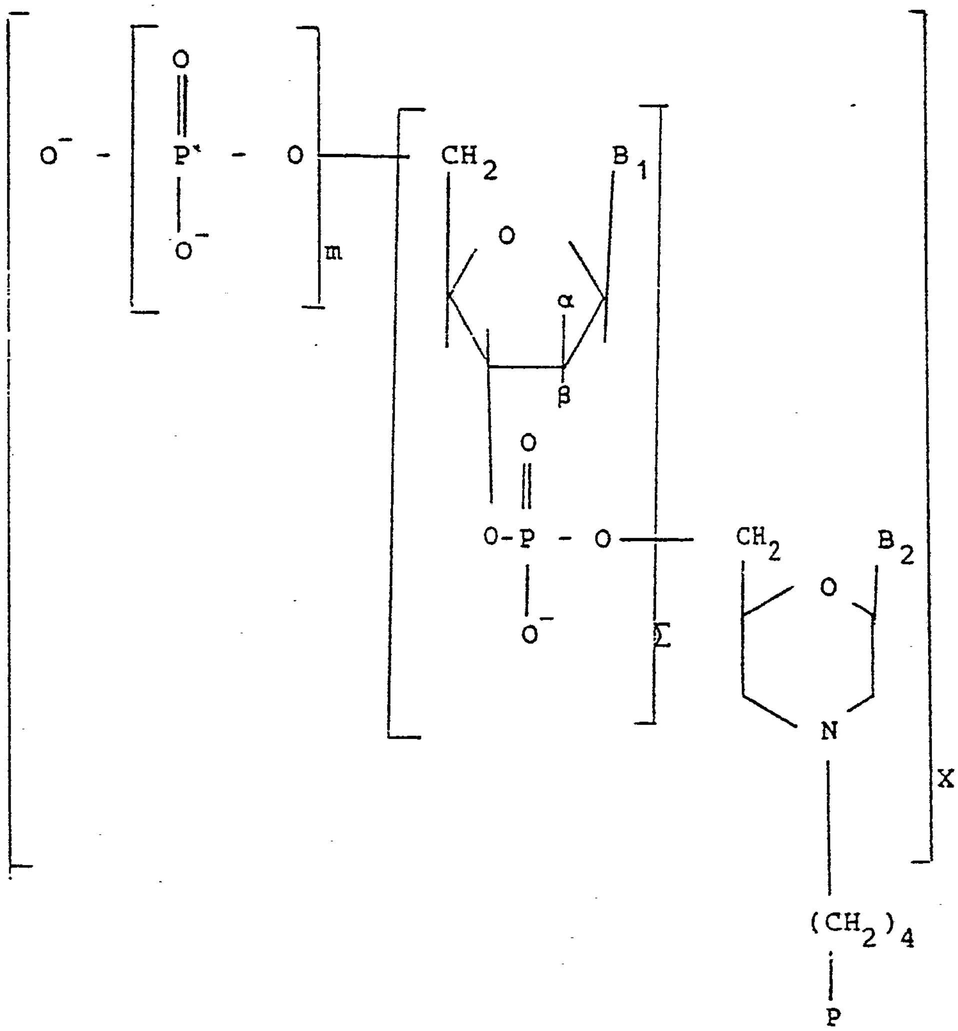

dans laquelle m, Σ, X, α, β, $B_1$, $B_2$ et P ont les significations indiquées à la revendication 5.

8. Conjugués de couplage selon la revendication 5, de formule VI suivante

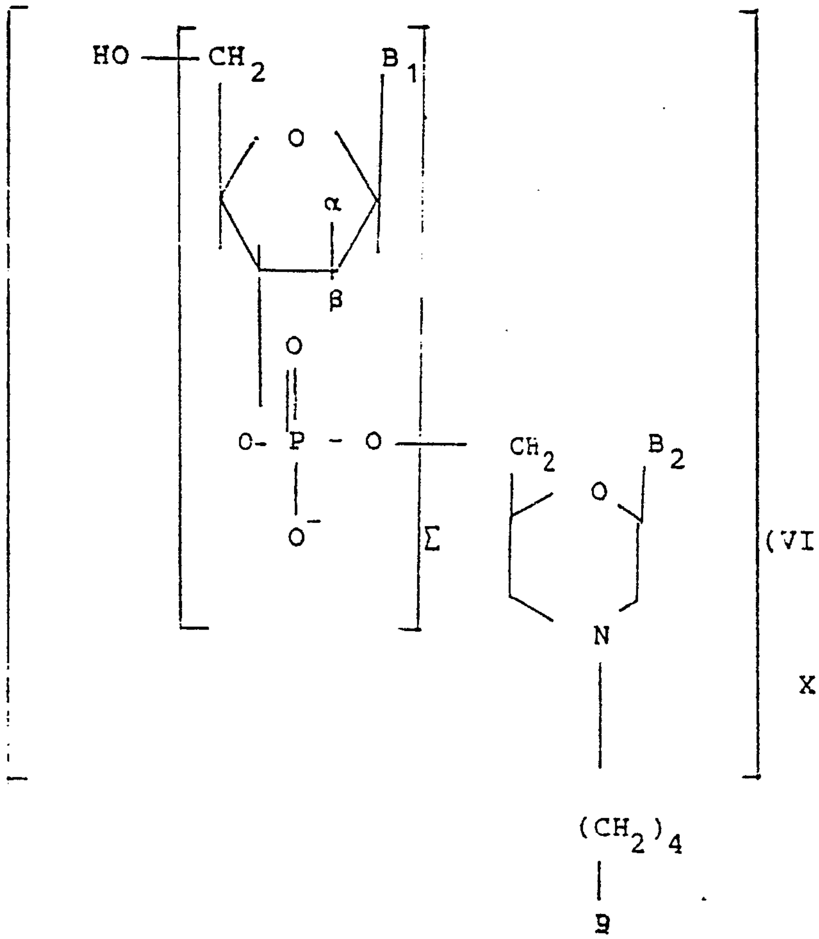

(VI)

dans laquelle $B_1$, $B_2$, α, β, Σ, X et P ont les significations indiquées à la revendication 5.

9. Composés de couplage selon la revendication 5, de formule VII suivante

(VII)

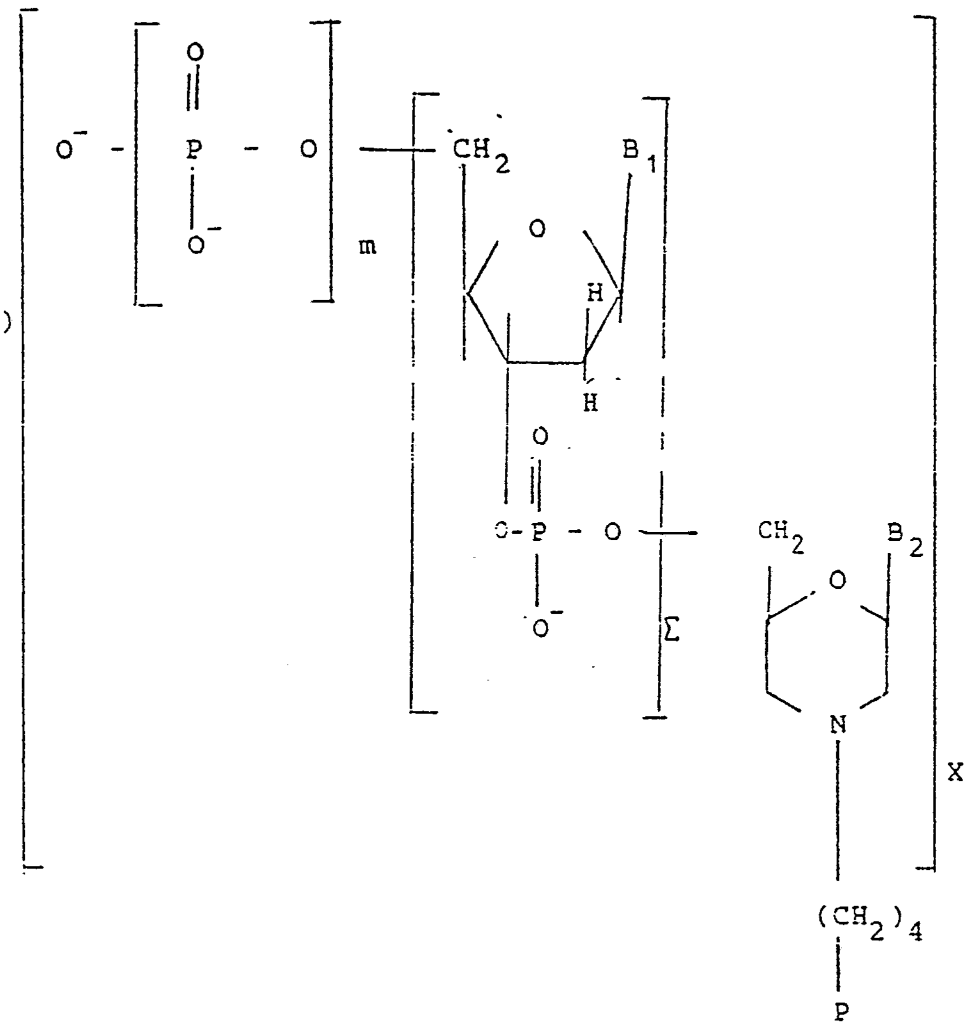

dans laquelle $B_1$, $B_2$, $\Sigma$, X, m et P ont les significations indiquées à la revendication 5.

10. Conjugués de couplage selon la revendication 5, de formule VIII suivante

(VIII) 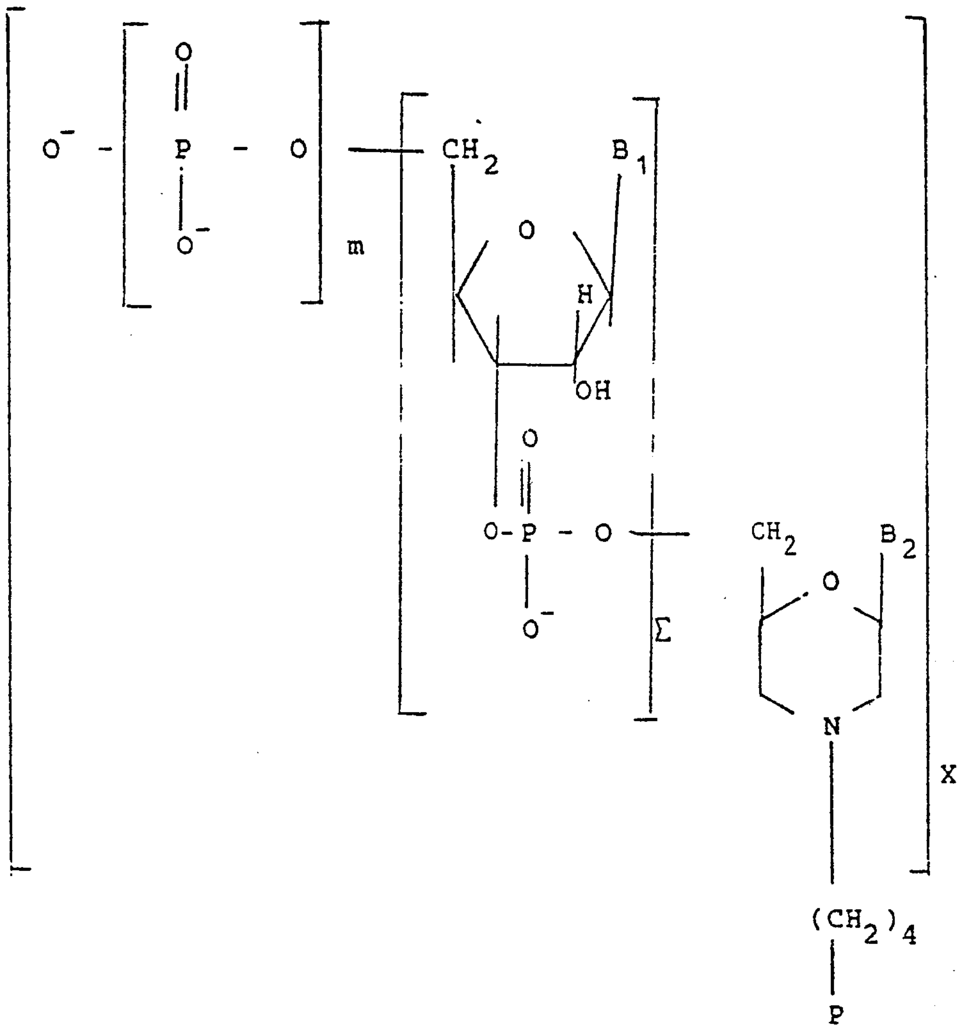

dans laquelle $B_1$, $B_2$, X, m, $\Sigma$ et P ont les significations indiquées à la revendication 5.

11. Conjugués de couplage selon la revendication 5, de formule IX suivante

(IX) 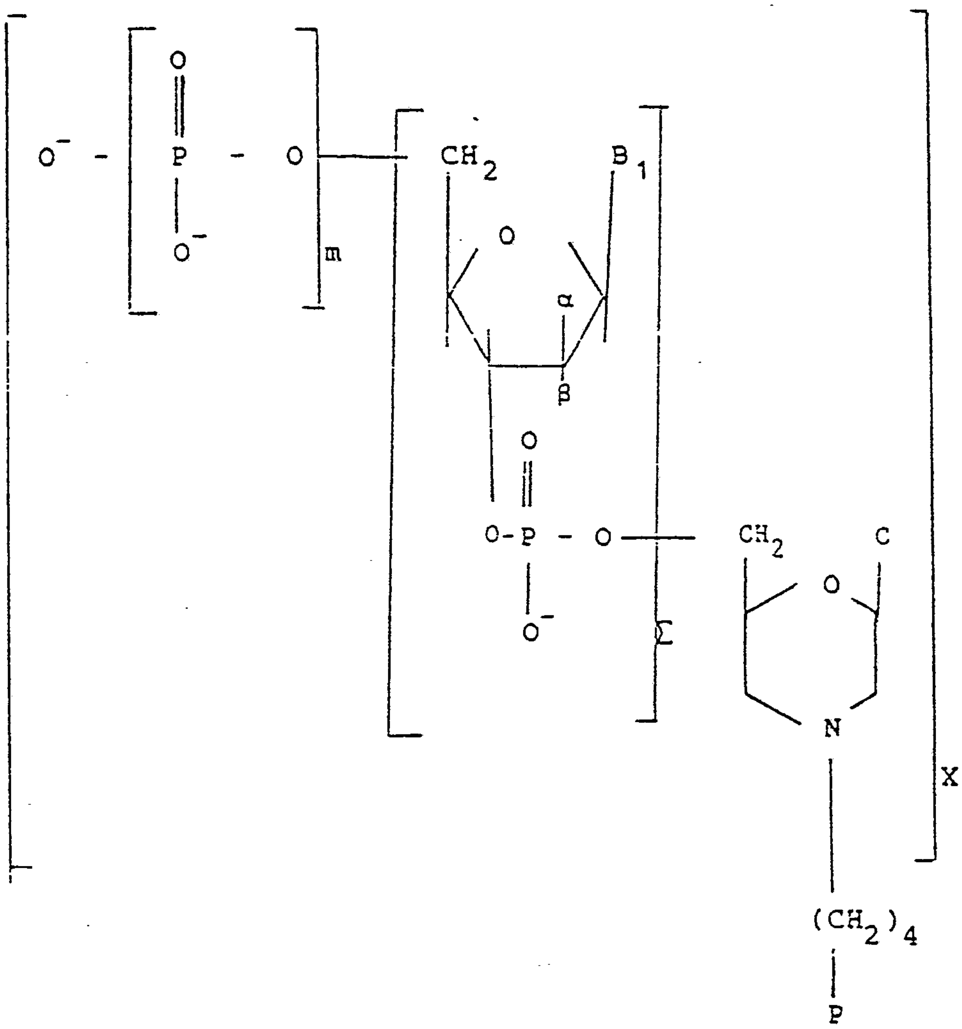

dans laquelle X, Σ, m, α, β, $B_1$ et P ont les significations indiquées à la revendication 5 et C représente la cytidine.

12. Conjugués de couplage selon la revendication 5, de formule XI suivante

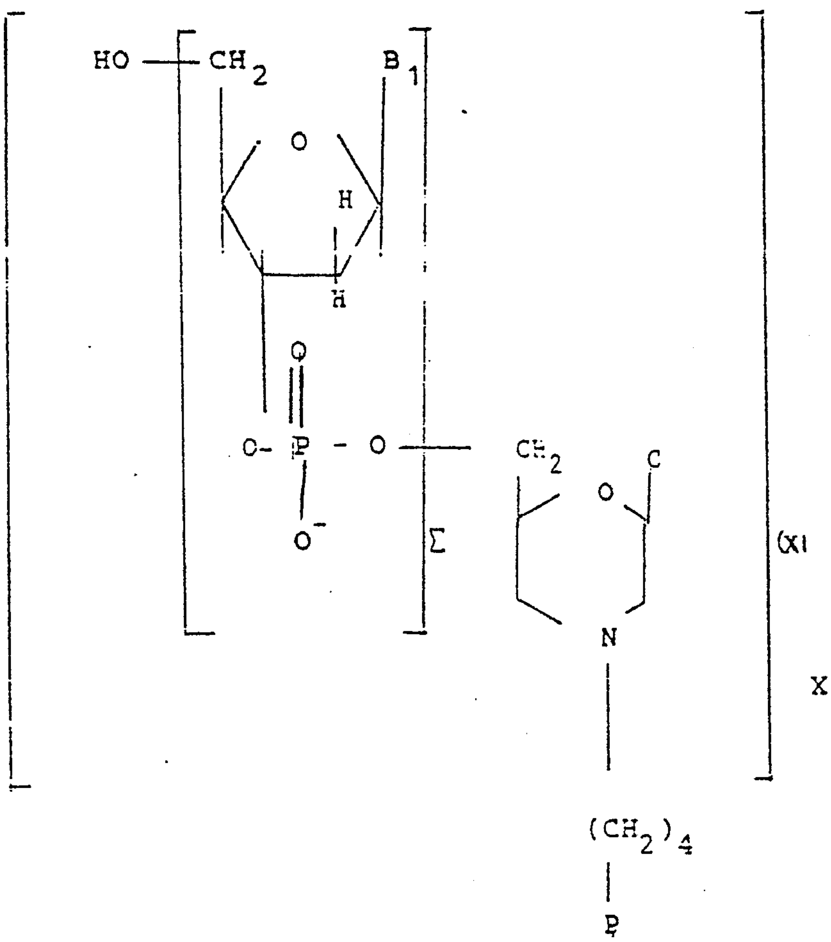

(XI)

dans laquelle $B_1$ et P ont la signification indiquée à la revendication 5, C représente la cytidine, Σ vaut 12 à 15 et X vaut 1 à 5, de préférence 1.

13. Conjugué de couplage selon les revendications 5 à 12, caractérisé en ce que P′ a un poids moléculaire d'environ 5 000 à environ 40 000, notamment d'environ 10 000 à environ 20 000, et avanatgeusement d'environ 15 000.

14. Conjugué de couplage selon la revendication 12 de formule XII suivante

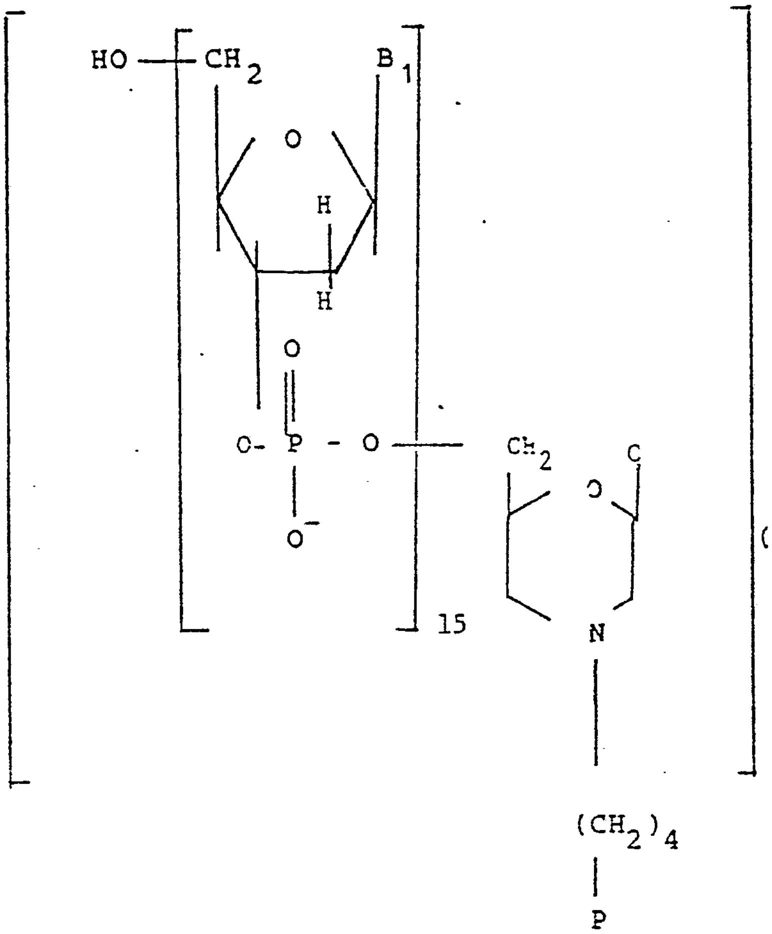

(XII)

dans laquelle
- P représente la poly (L) lysine de poids moléculaire d'environ 15 000,
- C représente la cytidine,
- Σ vaut 15, et
- l'enchaînement des bases $B_1$ est le suivant

3' TGTCATTAGTTTTAC 5'
et de formule XIII suivante

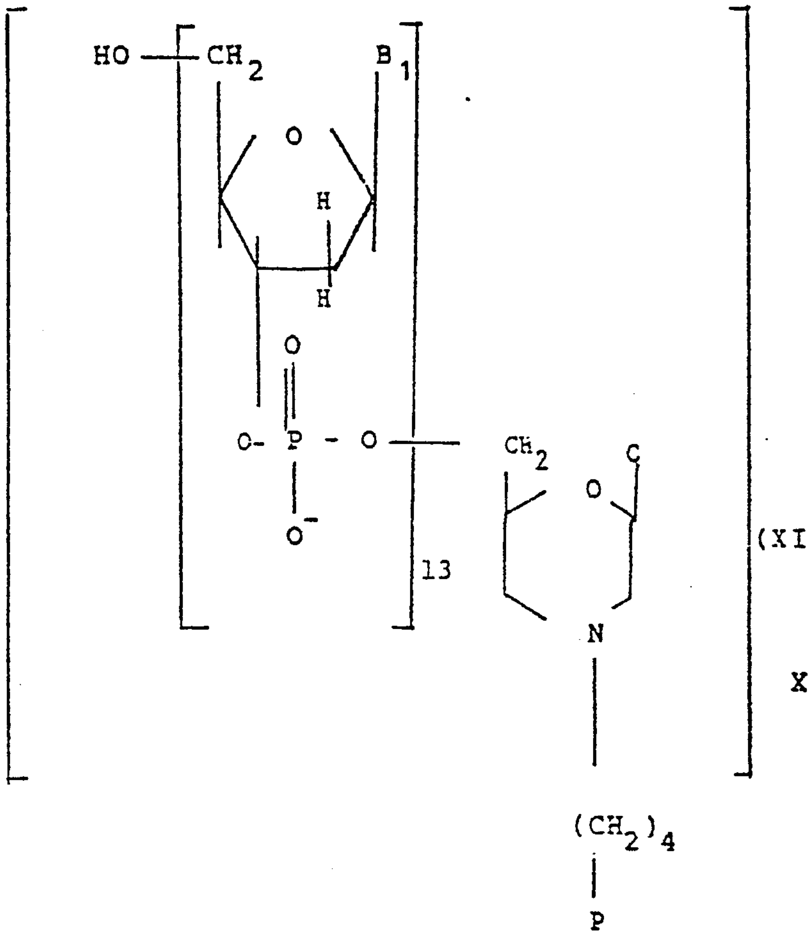

(XIII)

dans laquelle
- P représente la poly (L) lysine de poids moléculaire d'environ 15 000,
- C représente la cytidine,
- $\Sigma$ vaut 13 et
- l'enchaînement des bases $B_1$ est le suivant

$^{3'}$TTACACGGAGCAA$^{5'}$

15. Procédé de prépration des conjugués de couplage selon l'invention caractérisé en ce que
- on effectue la synthèse du composé de formule Ibis suivante

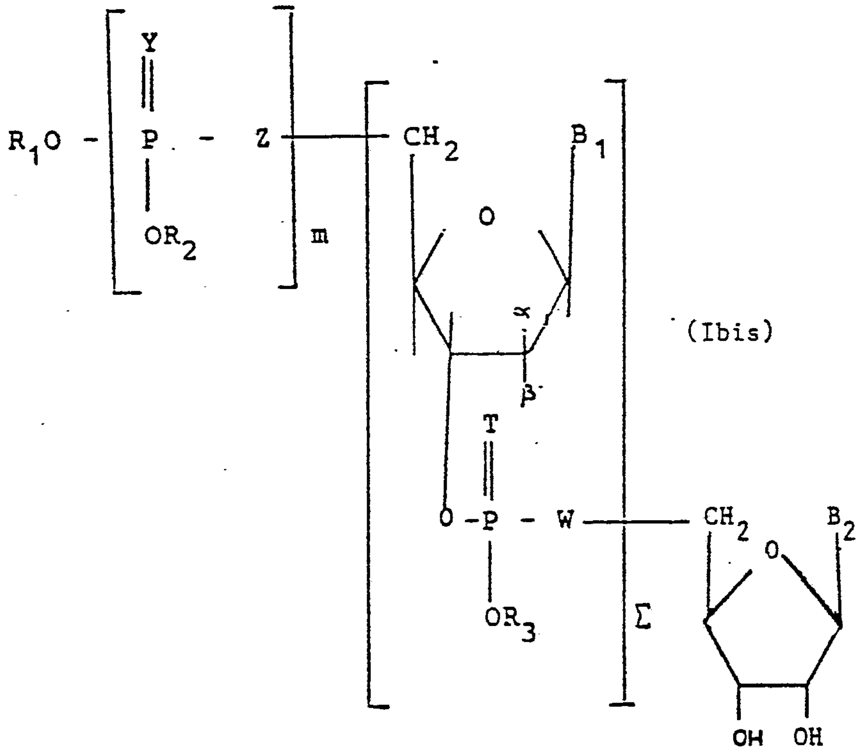

(Ibis)

dans laquelle

$R_1$, $R_2$, $R_3$, m, $\Sigma$, $B_1$, $B_2$, Y, Z, T, W, $\alpha$ et $\beta$ ont les significations indiquées à la revendication 1 ;

- on oxyde le composé de formule Ibis pour introduire deux fonctions aldéhyde sur les carbones en 2′ et 3′ de la dernière unité nucléosidique et obtenir le composé de formule Iter suivant

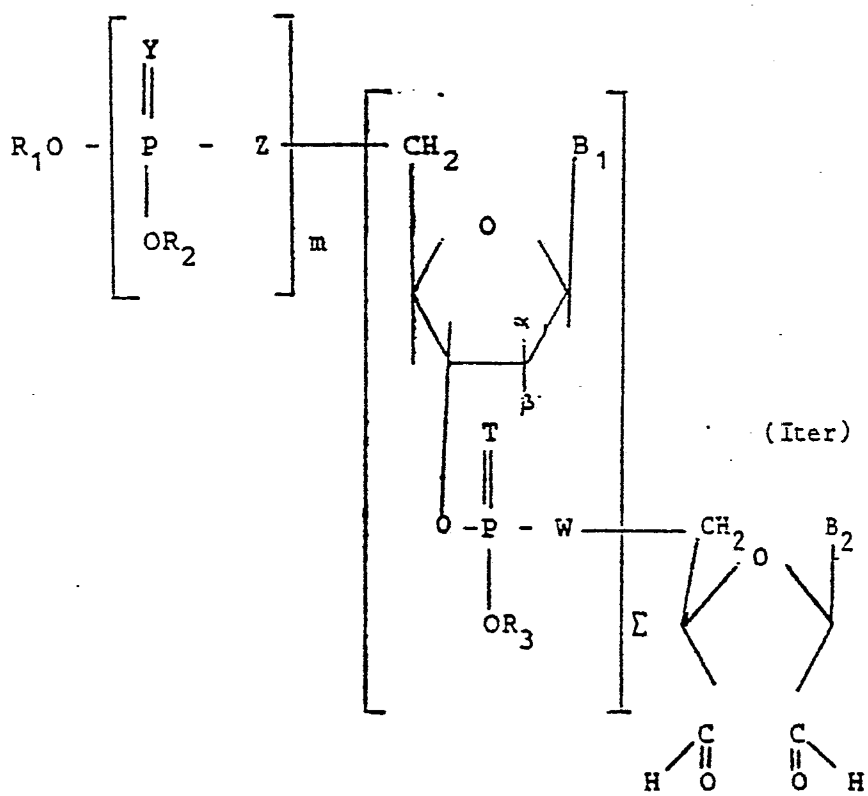

(Iter)

- on alcoyle de façon réductive le composé de formule Iter par un polypeptide P-$NH_2$ dont l'echaînement comprend au moins une séquence latérale dont l'extrémité est un groupe aminé, notamment $NH_2$, cette réaction ayant lieu entre les fonctions aldéhyde du composé (Iter) avec le groupe $NH_2$ du polypeptide

pour obtenir le composé de formule (I)

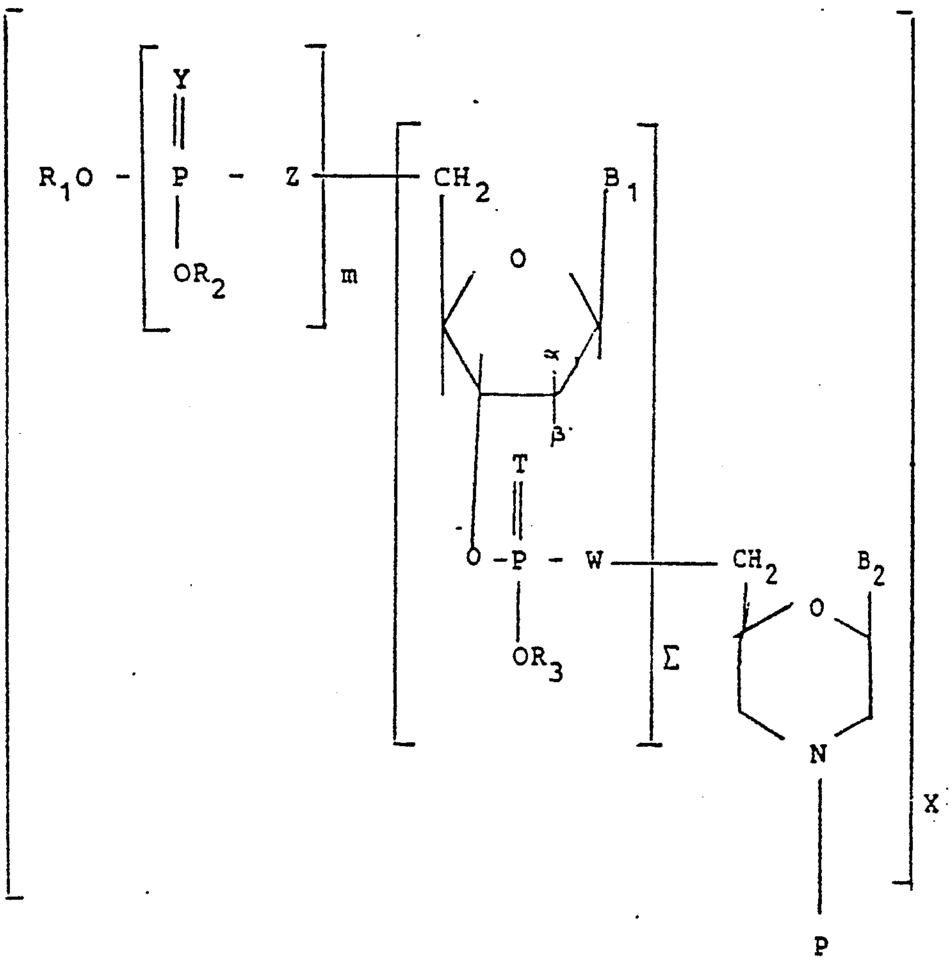

- on effectue le fractionnement entre les composés de couplage de formule I et les composés Iter et $PNH_2$ non couplés, notamment par filtration moléculaire ;

16. Procédé de préparation des conjugués de couplage selon la revendication 15 dans lequel le composé de formule Ibis

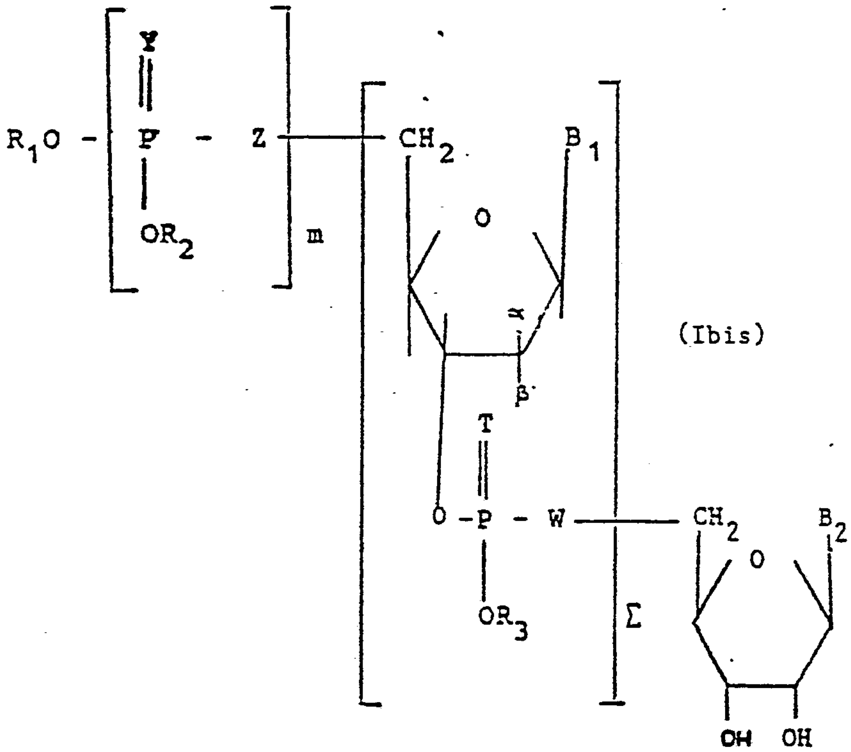

(Ibis)

dans laquelle $R_1$, $R_2$, $R_3$, Σ, m, $B_1$, $B_2$, Y, Z, T, W ont les significations indiquées à la revendication 1 et (α, β) représente (H, H) ou (OH, H)
est obtenu à partir du composé de formule Iquater

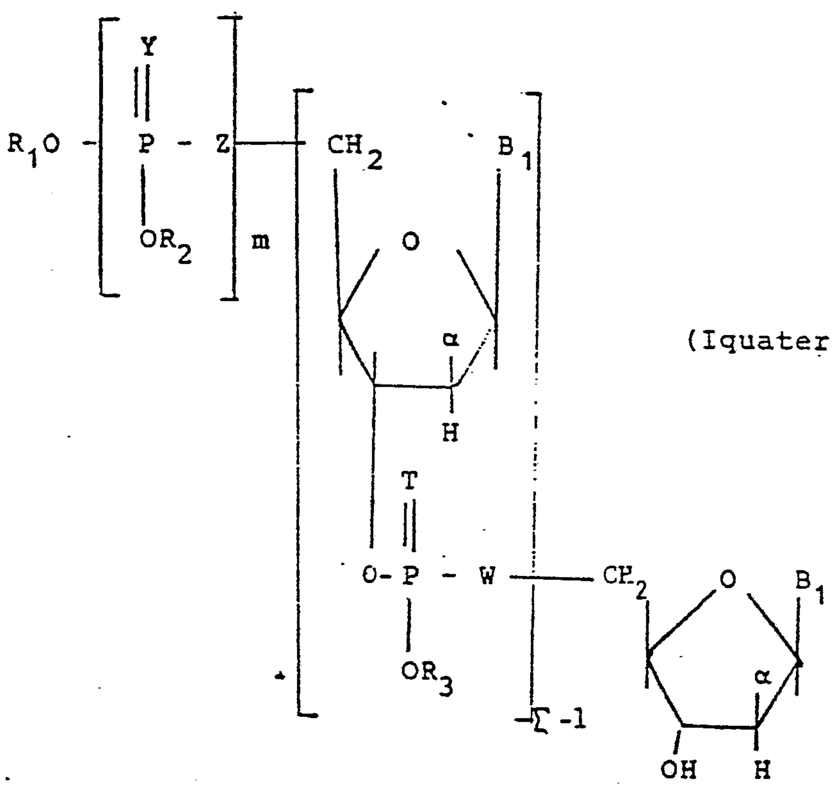

(Iquater)

dans lequel $R_1$, $R_2$, $R_3$, Σ, m, $B_1$, Y, Z, T, W ont les significations indiquées à la revendication 1 et α représente H ou OH, sur lequel on fait réagir le composé de formule

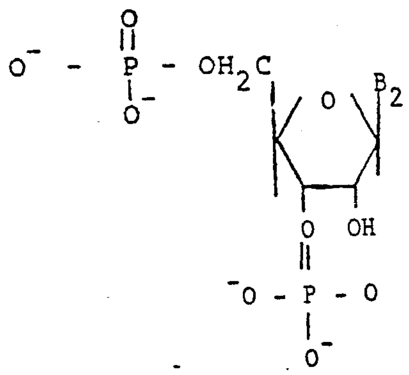

(Iquinquies)

dans lequel $B_2$ représente A, T, C, G ou U, et avantageusement C, en présence de RNA ligase de $T_4$ pour obtenir le composé de formule Isexies

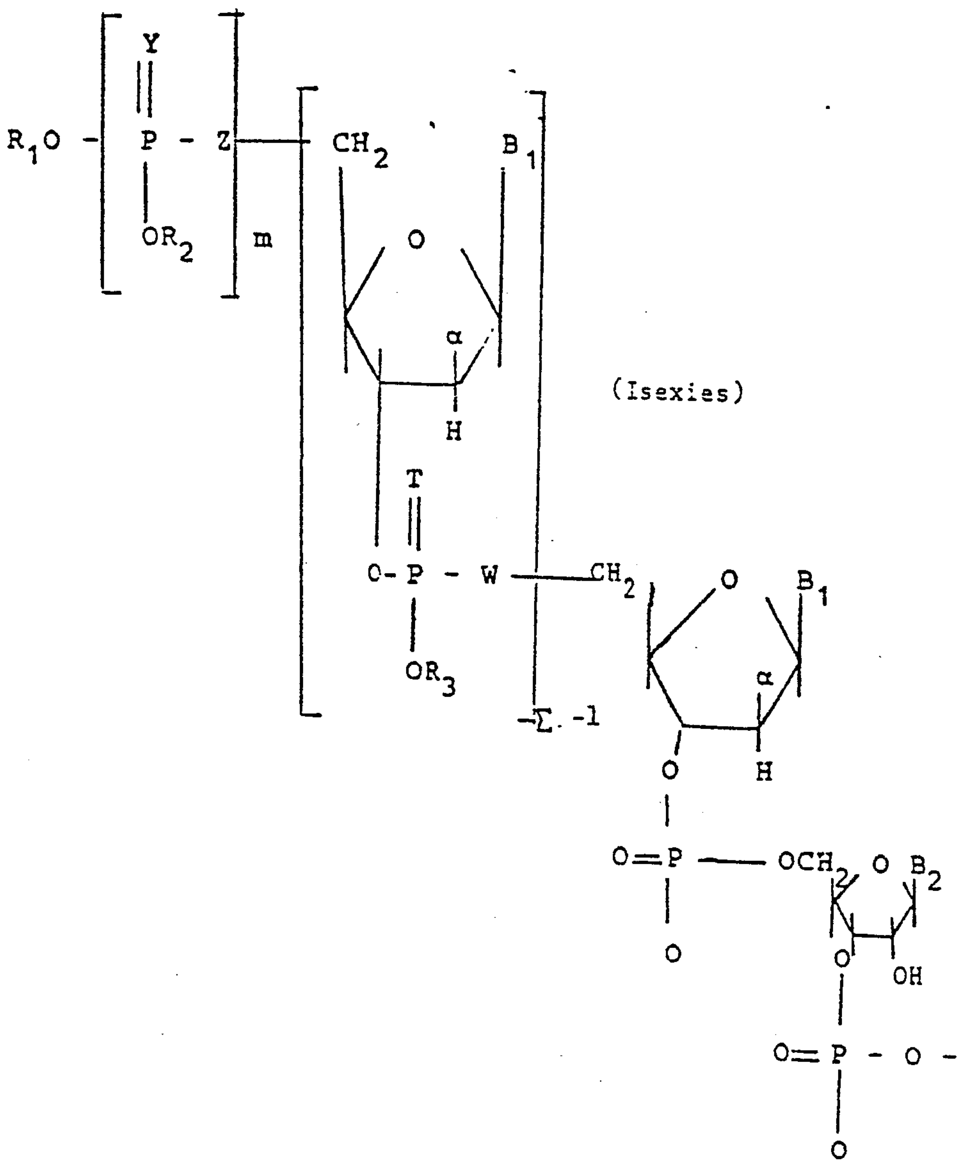

(Isexies)

- puis on effectue une filtration sur gel pour séparer le composé Isexies des composés Iquater et Iquinquies n'ayant pas réagi ;
- puis on fait agir la phosphatase alcaline bactérienne pour donner le composé de formule Ibis ou de formule (Ibis)',

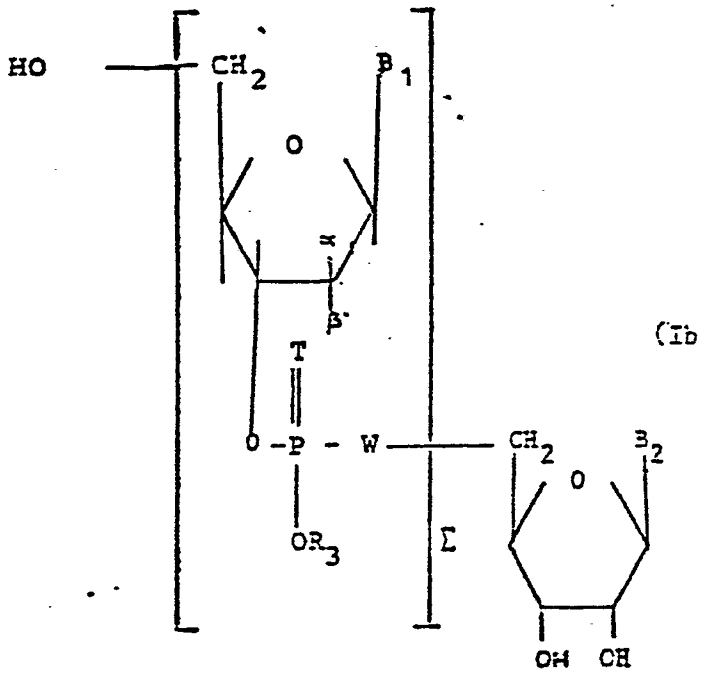

(Ibis)'

lorsque le groupe

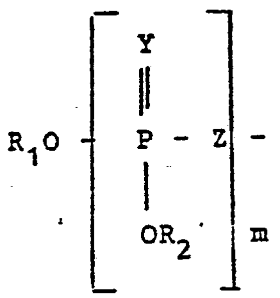

est le groupe

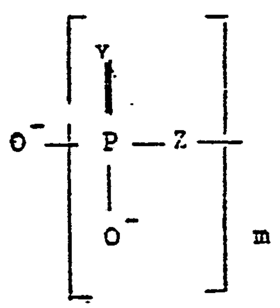

17. Procédé de préparation de conjugués de couplage selon la revendication 15, dans lequel le composé de formule Ibis est obtenu en phase solide à l'aide d'un support ribosylé sur lequel sont successivement additionnées des unités nucléotidiques.

18. Réactif biologique caractérisé en ce qu'il comprend au moins l'un des conjugués de couplage selon les revendications 1 à 14.

19. Procédé in vitro d'inhibition de l'expression d'un gène, notamment dans un milieu de culture cellulaire, à l'aide d'un au moins des conjugués de couplage selon les revendications 1 à 14.

1 SEQUENCE TERMINALE 5'

met ser val

5' AACAGTAATCAAAATGTCTGTT... 3'
TGTCATTAGTTTTAC

2 SEQUENCE INTERNE

640 650

5' ...AACATGAATGTGCCTCGTTCAGATA...3'
TTACACGGAGCAA

FIG.1

0263740

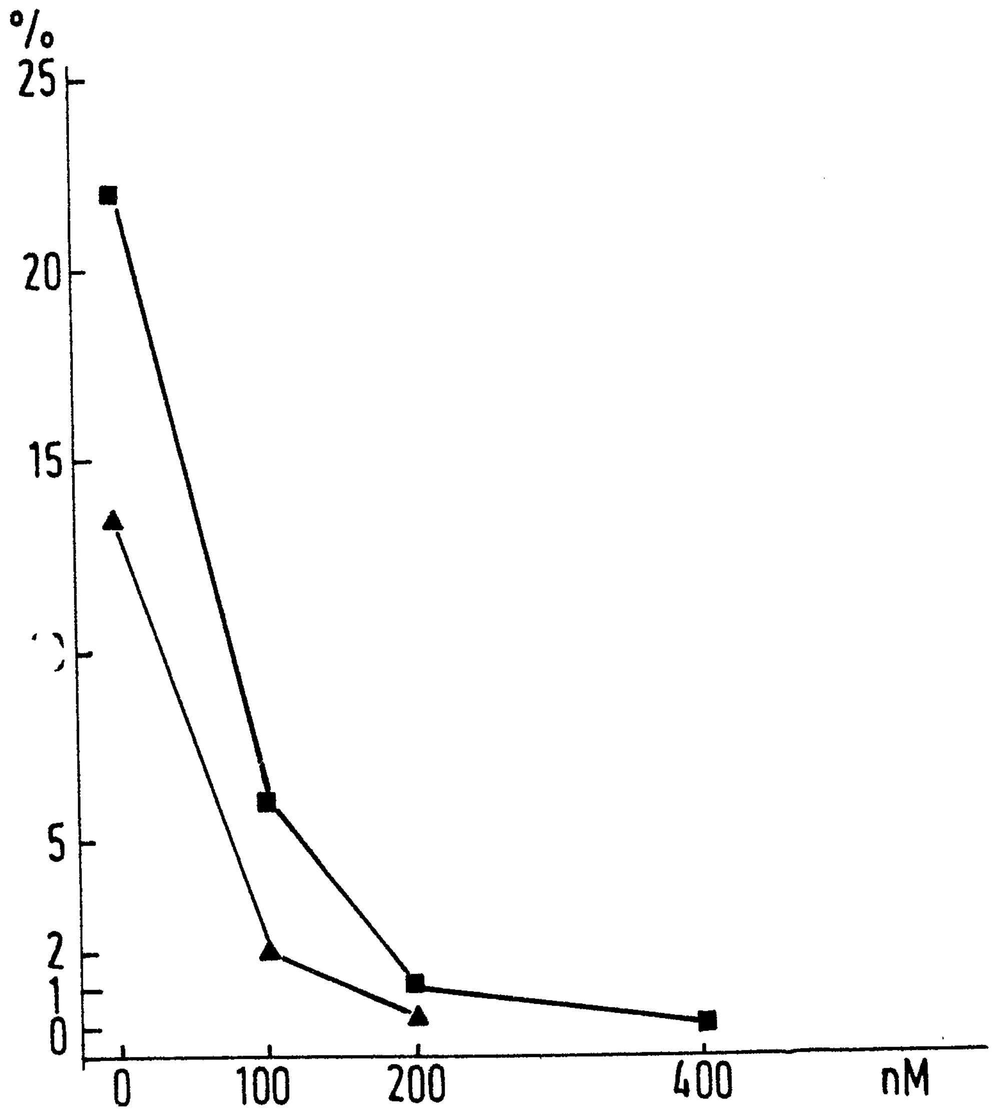


FIG.2

0263740

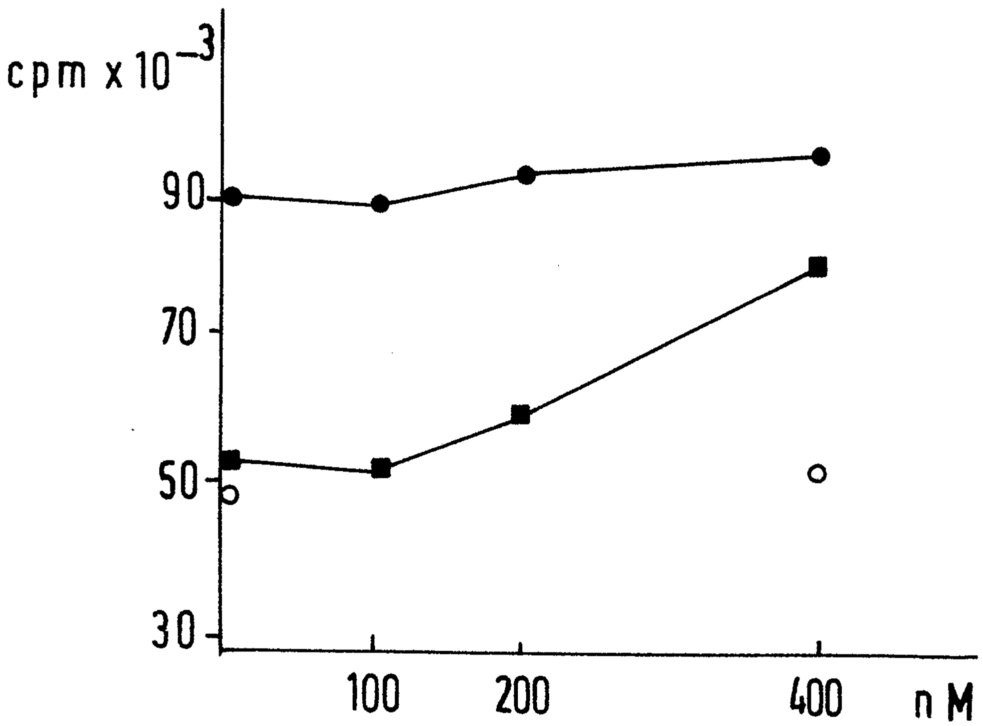


FIG.3

Office européen des brevets

# RAPPORT DE RECHERCHE EUROPEENNE

Numero de la demande

EP 87 40 2142

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | NUCLEIC ACIDS RESEARCH, vol. 11, no. 3, 1983, pages 659-669, IRL Press LTD, Oxford, GB; SHEUE-YANN CHENG et al.: "A versatile method for the coupling of protein to DNA: synthesis of alpha2-macroglobulin-DNA conjugates"<br>* Page 659 *<br>--- | 1 | C 07 K 9/00<br>C 07 K 15/18<br>A 61 K 37/10<br>G 01 N 33/549<br>C 12 N 1/38 |
| A | CHEMICAL ABSTRACTS, vol. 95, no. 3, 20 juillet 1981, page 755, résumé no. 25594w, Columbus, Ohio, US; V. KIRVELIENE et al.: "Synthesis and some properties of uridylyl-(PO)-amino acids (peptides)", & AKTUAL. PROBL. RAZVIT. NAUCHN. ISSLED. MOLODYKH UCH. SPETS. VIL'NYUS. GOSUNIV. IM. V. FAK. 1980, 32-3<br>* Résumé *<br>----- | 1 | |
| | | | **DOMAINES TECHNIQUES RECHERCHES (Int. Cl.4)** |
| | | | C 07 K 9/00 |

Le présent rapport a été établi pour toutes les revendications

| Lieu de la recherche | Date d'achèvement de la recherche | Examinateur |
|---|---|---|
| LA HAYE | 17-12-1987 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)